# EUROPEAN PATENT SPECIFICATION

(11) **EP 1 453 545 B1**
(45) Date of publication and mention of the grant of the patent: **18.04.2007**
(21) Application number: 02792256.6
(22) Date of filing: 30.10.2002
(51) Int. Cl.: A61K 47/48, A61K 9/00, A61K 31/07, A61P 11/00

(54) **WATER-SOLUBLE POLYMER CONJUGATES OF RETINOIC ACID**
WASSERLÖSLICHE POLYMERKONJUGATE VON RETINOESÄURE
CONJUGUES POLYMERES HYDROSOLUBLES D'ACIDE RETINOIQUE

(30) Priority: 30.10.2001 US 335043 P
(43) Date of publication of application: 08.09.2004
(73) Proprietor: Nektar Therapeutics Al, Corporation, Huntsville, AL 35806-2902 (US)
(72) Inventor: BENTLEY, Michael, David, Huntsville, AL 35801 (US); ZHAO, Xuan, Huntsville, AL 35803 (US); LEACH, Chester, El Granada, CA 94018 (US); KUO, Mei-Chang, Palo Alto, CA 94301 (US); CHARAN, Chatan, K., San Jose, CA 95148 (US)
(74) Representative: Baldock, Sharon Claire
(86) International application number: PCT/US2002/036421
(87) International publication number: WO 2003/037385

(56) References cited:
- WO-A-01/68081
- WO-A-97/33552
- US-A- 4 371 673
- US-A- 5 210 133
- US-B1- 6 277 890
- BROOKS A D ET AL: "INHALED AEROSOLIZATION OF ALL-TRANS-RETINOIC ACID FOR TARGETED PULMONARY DELIVERY" CANCER CHEMOTHERAPY AND PHARMACOLOGY, SPRINGER VERLAG, BERLIN, DE, vol. 46, no. 6, October 2000 (2000-10), pages 313-318, XP001018001 ISSN: 0344-5704

## Description

### FIELD OF THE INVENTION

This invention relates to water-soluble polymer conjugates of biologically active retinoids, and in particular, to compositions of water-soluble retinoid polymer conjugates that are suitable for delivery to the lung by inhalation. Methods for preparing and administering such conjugates are also provided.

### BACKGROUND OF THE INVENTION

Retinoids, such as retinol, retinoic acid, and retinyl esters, are all considered forms of vitamin A. Several derivatives of vitamin A exist such as beta carotene, retinal, retinol, all trans retinoic acid, 9-cis retinoic acid and 13-cis retinoic acid. Vitamin A is considered an essential micronutrient, and a deficiency of vitamin A can have detrimental effects, such as hornification (hyperkeratosis) of the mucous membranes, especially those of the respiratory system. In severe cases, a deficiency of vitamin A can lead to increased susceptibility to bronchial infections and even blindness (McDowell, E.M., et al., Cell Pathol., 45:197-219, 1984). Fortunately, in most cases, intake of vitamin A can lead to reversal of these vitamin A deficiency-induced conditions. Interestingly, diseases of the mucous membranes of the respiratory system such as acute and chronic bronchitis, emphysema, and even certain types of cancer, not induced by a deficiency of vitamin A, have also been successfully treated or prevented by the systemic administration of vitamin A, usually in high doses. In fact, retinoids have been used successfully in the treatment of a number of conditions including skin disorders such as acne, and cancers such as acute promyleocytic leukemia, lung cancer, prostate cancer, and breast cancer.

Unfortunately, due to the high dosages required for effective treatment, the retinoids have toxic side effects that can be devastating and potentially fatal. These adverse effects include hyperlipidemia, hypercalcemia, and skin, liver, and central nervous system toxicity. Additionally, most of the naturally occurring forms of vitamin A, such as the all trans and cis isomers of retinoic acid, are lipophilic, meaning that they are insoluble in water. Not only does this insolubility contribute to the need to administer high doses of retinoids in order to be efficacious, but water insoluble drugs such as these are extremely difficult to formulate. Moreover, as a result of their insolubility in water, drugs such as retinoic acid often possess extremely low bioavailabilities.

Thus, there exists a need for new soluble forms and formulations of retinoids, preferably water soluble forms of retinoids such as retinoic acid that can be readily formulated into therapeutic compositions, and that (i) are not hampered by the problems that plague the naturally occurring forms of vitamin A such as low bioavailability and high toxicity, (ii) do not require the addition of alkylamines or surfactants or detergents to solubilize the retinoid, and (iii) may, in certain instances, be delivered to the lung by inhalation, particularly for the localized treatment of diseases of the respiratory tract.

### SUMMARY OF THE INVENTION

Generally, the present invention provides new water-soluble derivatives of retinoic acid that, based upon their aqueous solubility, are much easier to formulate, process, and deliver than their unmodified retinoid counterparts. Additionally, the modified retinoids of the invention are particularly well suited for administration by inhalation. In fact, the inventors have discovered that in addition to the above-mentioned advantages associated with the increased water solubility of the herein provided retinoid derivatives, the compounds of the invention, when administered to the lung, can depot in lung tissue, meaning that such compounds are particularly advantageous for localized delivery to the lung for treating chronic obstructive pulmonary diseases such as emphysema. Thus, the administration of such compounds directly to the lung, coupled with the increased water solubility of the herein provided retinoids, may allow administration of smaller doses of the retinoid derivatives of the invention to achieve therapeutically useful levels, thereby reducing their systemic toxicity.

In one aspect, the present invention provides a polymer retinoid conjugate composition suitable for pulmonary administration, wherein the composition comprises a retinoid covalently bonded to a water-soluble and non-peptidic polymer.

The water-soluble and non-peptidic polymers for use in forming a conjugate of the invention are poly(alkylene glycols), poly(oxyethylated polyol), poly(olefinic alcohol), poly(vinylpyrrolidone), poly(hydroxyalkylmethacrylanude), poly(hydroxyalkylmethacrylate), poly(α-hydroxy acid), poly(vinyl alcohol), polyphosphazene, polyoxazoline, poly(N-acryloylmorpholine), and copolymers, terpolymers, and mixtures thereof. In one particularly preferred embodiment of the invention, the polymer is a polyethylene glycol (PEG). The polymer portion of the conjugate may possess any of a number of geometries including linear, branched, forked or dumbell structures.

In one embodiment of the invention, the inhaleable composition comprises a conjugate prepared from a lipophilic retinoid selected from the group consisting of 13-cis retinoic acid, all trans retinoic acid, 9-cis retinoic acid, 11-cis retinoic acid, and retinol.

The compositions of the invention may be liquid or may be dry powder formulations.

Also encompassed by the invention is an aerosol of a polymer retinoid conjugate composition as described above.

In one embodiment of the invention, the aerosol composition is contained in an inhaler device.

In yet another embodiment, the polymer conjugate composition further comprises a free radical scavenger.

In an alternative embodiment, provided is a spray dried composition of a polymer retinoid conjugate.

According to yet another embodiment, the water soluble and non-peptidic polymer is covalently bonded to the retinoid via a hydrolytically unstable linkage. Preferred linkages include ester, thiolester (-C(O)-S) and amide.

In yet another embodiment, the polymer retinoid conjugate composition is absent an agent necessary for solubilizing the retinoid in a carrier vehicle.

A method is provided for administering a water-soluble form of a retinoid to a mammalian subject in need thereof. The method includes the steps of (i) providing a polymer retinoid conjugate composition as described herein, (ii) aerosolizing the composition from (i) to form an aerosolized composition, and (iii) administering to the subject by inhalation the aerosolized composition for localized deposition in the lung of the subject.

In accordance with yet another aspect of the invention, a method is provided for providing a water-soluble retinoid composition for administration to the lung of a subject in need thereof. The method includes the steps of (i) covalently bonding a retinoid to a water soluble and non-peptidic polymer as defined previously to form a water soluble polymer retinoid conjugate, (ii) providing a pharmaceutically acceptable composition comprising the conjugate from (i) and (iii) aerosolizing the composition. In a preferred embodiment, the subject is suffering from a chronic obstructive pulmonary disease such as emphysema.

In yet another aspect, the invention provides particular polymer retinoid conjugates.

In one embodiment, the invention provides a polymer retinoid conjugate comprising a water soluble and non-peptidic polymer covalently attached to the carbonyl carbon of retinoic acid to form a hydrolytically degradable linkage.

According to one specific embodiment of the polymer retinoid conjugate per se, if the polymer is a linear polyethylene glycol and the linkage is an ester linkage, then the molecular weight of the polyethylene glycol is at least about 2,000 daltons. According to yet another embodiment of the polymer conjugate per se, if the polymer is a linear end-capped polyethylene glycol, the retinoid is ATRA, and the linkage is an amide linkage, then the polyethylene glycol has a molecular weight of at least about 5,000 daltons.

In yet another embodiment of the invention, provided is a polymer retinoid conjugate having the following structure: 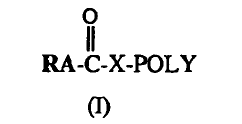 wherein RA together with the carbonyl group represents a retinoic acid moiety,
X is a heteroatom; and
POLY is a water soluble, non-peptidic polymer.

In a specific embodiment of structure (I), X is a heteroatom selected from O, N-H, and S, wherein (i) if the POLY is a linear polyethylene glycol and X is an O, then the molecular weight of the POLY is at least about 2,000 daltons, and (ii) if POLY is a linear end-capped polyethylene glycol, and RA together with the adjacent carbonyl represents an ATRA moiety, and X is a N-H, then POLY has a molecular weight of at least about 5,000 daltons.

In a preferred embodiment, a conjugate of the invention will contain a hydrolytically degradable linkage, such as an ester linkage, between the retinoic acid moiety and the polymer. Thus, in such an embodiment, the polymer conjugate is considered to be a prodrug, meaning that the hydrolyzable linkage can hydrolyze to liberate the unmodified parent retinoid.

Particularly preferred conjugates will possess the following generalized structures, wherein X and POLY are as defined above.

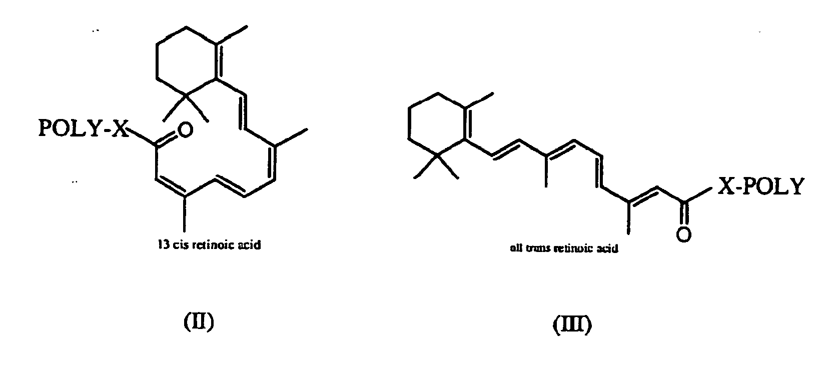

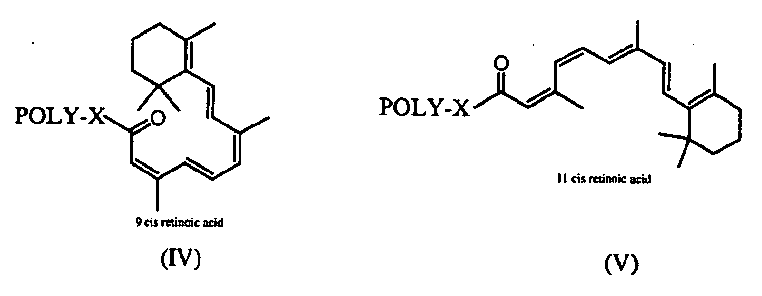

The POLY portion of a conjugate of the invention may be linear, such as methoxy PEG, branched (or multi-armed), or forked. In particular embodiments of the invention wherein the polymer is linear, the conjugate may incorporate a heterobifunctional or a homobifunctional polymer. A conjugate of a heterobifunctional polymer is one wherein one terminus of the polymer is attached to the retinoid and the other terminus is functionalized with a different moiety. A conjugate of a homobifunctional polymer possesses a structure wherein each end of a linear polymer is covalently attached to a retinoid, typically by an identical linkage.

The invention also provides a method of forming a polymer conjugate of a retinoic acid. The method includes the steps of providing a water soluble and non-peptidic polymer having a terminus that is a functional group that is reactive with a carboxylic acid or an acid halide group, such as hydroxyl, amino or thiol. Such a polymer is then reacted with retinoic acid or an activated form of retinoic acid such as the corresponding acid halide to form a polymer retinoid conjugate having, for example, an ester or thiolester or amide linkage between the polymer backbone and the retinoic acid moiety.

A polymer-retinoid conjugate of the invention, when aerosolized and administered via inhalation, is particularly useful in the treatment of emphysema.

These and other objects and features of the invention will become more fully apparent when considered in view of the detailed description which follows.

### BRIEF DESCRIPTION OF THE FIGURES

**FIG. 1** graphically illustrates the rate of hydrolysis of a representative polymer conjugate of retinoic acid in buffer. The conjugate contains an ester linkage coupling the retinoid moiety to the polymer. The hydrolysis study is described in Example 9.
**FIG. 2** graphically illustrates the rate of hydrolysis of a representative polymer conjugate of retinoic acid in rat serum. The conjugate contains an ester linkage coupling the retinoid to the polymer; the hydrolysis study is described in Example 10.
**FIG. 3** demonstrates the concentrations of ATRA and PEG-5kD-ATRA ester conjugate in rat lung following intratracheal administration as described in Example 11.

### DETAILED DESCRIPTION OF THE INVENTION

In one respect, the design, synthesis, characterization and formulation of various representative PEG-retinoic acid conjugates have been optimized for pulmonary delivery to the lung. Heretofore, the preparation and formulation of PEG-retinoic acid conjugates for delivery to the lung has not been demonstrated. The conjugates of the invention offer many advantages, including most notably, the water-soluble nature of the compositions and conjugates of the invention. Thus, the polymer conjugates provided herein are much easier to formulate into administrable therapeutic compositions when compared to their unmodified lipophilic parent retinoid counterparts. For instance, previous compositions of retinoids for inhalation therapy have been limited to metered dose inhaler compositions requiring the addition of an alkyl amine to the chlorofluorocarbon solvent in order to solubilize the retinoid (see for example, Tong, W., and Warrell, R., U.S. Patent No. 6,251,941). In contrast, the present retinoid conjugates, by virtue of their aqueous solubility, can be administered using any of a number of delivery vehicles without the need for additional solubilizing agents or emulsifiers (e.g., CREMAPHOR® or MOLECUSOL®), many of which are undesirable for administration to the lung.

Moreover, in forming certain spray dried compositions of the retinoid conjugates of the invention, the inventors were able to overcome drawbacks faced during the spray drying of unmodified retinoic acid, namely, sublimation of the low melting compound in the spray dryer. Thus, the covalent attachment of a polymer chain to retinoic acid was sufficient to raise both the melting and boiling points of retinoic acid to thereby make spray drying a much more attractive process, in terms of both yield and efficiency.

Lastly, in-vivo data indicates that the retinoid conjugates of the invention provide sustained levels of retinoic acid in the lungs - that is to say, the retinoic acid conjugates appear to depot in the lung to at least a measurable degree rather than rapidly absorbing through the lung tissue into the systemic circulation, which in turn leads to a reduction in systemic toxicity when compared to i.v. injections. Moreover, plasma data indicates that the polymer conjugates of the invention are retained in the lung to a much greater degree than the corresponding unmodified or non-polymer conjugated retinoid. That is to say, the unmodified retinoid absorbs relatively rapidly through the lung tissue into the circulation, while the conjugate is significantly retained in the lung, further demonstrating the advantages of a polymer-retinoid conjugate when compared to the unmodified parent in the localized treatment and prevention of conditions of the respiratory tract such as emphysema.

### I. Definitions

The following terms as used herein have the meanings indicated.

As used in the specification, and in the appended claims, the singular forms "a", "an", "the", include plural referents unless the context clearly dictates otherwise.

The terms "functional group", "active moiety", "reactive site", "chemically reactive group" and " chemically reactive moiety" are commonly used in the art and herein to refer to distinct, definable portions or units of a molecule. These terms are somewhat synonymous in the chemical arts and are used herein to indicate the portions of molecules that perform some function or activity and are reactive with other molecules. The term "active," or "reactive" when used in conjunction with a functional group, is intended to include those functional groups that react readily with electrophilic or nucleophilic groups on other molecules, in contrast to those groups that require strong catalysts or highly impractical reaction conditions in order to react (i.e., "non-reactive" or "inert" groups). For example, as would be understood in the art, the term "active ester" would include those esters that react readily with nucleophilic groups such as amines. Exemplary active esters include N-hydroxysuccinimidyl esters or 1-benzotriazolyl esters. Typically, an active ester will react with an amine in aqueous medium in a matter of minutes, whereas certain esters, such as methyl or ethyl esters, require a strong catalyst in order to react with a nucleophilic group. As used herein, the term "functional group" is meant to include protected forms.

The term "protected functional group" or "protecting group" or "protective group" refers to the presence of a moiety (i.e., the protecting group) that prevents or blocks reaction of a particular chemically reactive functional group in a molecule under certain reaction conditions. The protecting group will vary depending upon the type of chemically reactive group being protected as well as the reaction conditions to be employed and the presence of additional reactive or protecting groups in the molecule, if any. Protecting groups known in the art can be found in Greene, T.W., *et al*., PROTECTIVE GROUPS IN ORGANIC SYNTHESIS. 3rd ed., John Wiley & Sons, New York, NY (1999).

The term "linkage" or "linker" (L) is used herein to refer to an atom or a collection of atoms used to link, preferably by one or more covalent-bonds, interconnecting moieties such as two polymer segments or a terminus of a polymer and a reactive functional group present on a bioactive agent, such as retinoic acid. A linker of the invention may be hydrolytically stable or may include a physiologically hydrolyzable or enzymatically degradable linkage.

A "physiologically hydrolyzable" or "hydrolytically degradable" bond is a weak bond that reacts with water (i.e., is hydrolyzed) under physiological conditions. The tendency of a bond to hydrolyze in water will depend not only on the general type of linkage connecting two central atoms but also on the substituents attached to these central atoms. Exemplary hydrolytically unstable or degradable linkages include but are not limited to carboxylate ester, phosphate ester, thiolester, anhydrides, acetals, ketals, acyloxyalkyl ether, imines, orthoesters, peptides and oligonucleotides. Illustrative hydrolysis rates in both buffer and serum for representative conjugates of the invention are provided in Examples 9 and 10.

A "hydrolytically stable" linkage or bond refers to a chemical bond, typically a covalent bond, that is substantially stable in water, that is to say, does not undergo hydrolysis under physiological conditions to any appreciable extent over an extended period of time. Examples of hydrolytically stable linkages include but are not limited to the following: carbon-carbon bonds (e.g., in aliphatic chains), ethers, amides, urethanes, and the like. Generally, a hydrolytically stable linkage is one that exhibits a rate of hydrolysis of less than about 1-2% per day under physiological conditions. Hydrolysis rates of representative chemical bonds can be found in most standard chemistry textbooks, or alternatively, hydrolysis rates of a given compound can be measured using standard techniques known in the art.

An "enzymatically unstable" or "enzymatically degradable" linkage is a linkage that can be degraded by one or more enzymes.

"PEG" or polyethylene glycol, as used herein, is meant to encompass any water-soluble poly(ethylene oxide). Most typically, PEGs for use in the present invention will contain the following structure, "-CH₂CH₂O(CH₂CH₂O)ₙCH₂CH₂-, wherein the terminal groups or actual architecture of the overall PEG moiety may vary. One commonly employed PEG is end-capped PEG, wherein one terminus of the PEG is capped with a relatively inert group, typically an alkoxy group such as methoxy (-OCH₃), while the other terminus is a hydroxyl group that can then be subjected to chemical modification. The term "PEG" includes poly(ethylene glycol) in any of its linear, branched or multi-arm forms, including alkoxy PEG, bifunctional PEG, forked PEG, branched PEG, pendent PEG, or PEG with degradable linkages therein. Specific PEG forms for use in preparing the retinoid conjugates of the invention, such as branched, linear, forked PEGs, and the like, will be described in greater detail below.

"Nominal average molecular weight" in the context of a hydrophilic, non-peptidic polymer of the invention such as PEG, refers to the mass average molecular weight of polymer, typically determined by size exclusion chromatography, light scattering or intrinsic velocity in 1,2,4-trichlorobenzene. The polymers of the invention are typically polydisperse, possessing a low polydispersity value of less than about 1.05.

The term "alkyl", "alkenyl", and "alkynyl" refers to hydrocarbon chains typically ranging from about 1 to about 12 carbon atoms in length, preferably 1 to about 6 atoms, and includes straight and branched chains. Unless otherwise noted, the preferred embodiment of any alkyl referred to herein is C1-C6alkyl (e.g., methyl or ethyl).

"Cycloalkyl" refers to a saturated or unsaturated cyclic hydrocarbon chain, including bridged, fused, or spiro cyclic compounds, preferably comprising 3 to about 12 carbon atoms, more preferably 3 to about 8.

The term "substituted alkyl", "substituted alkenyl", "substituted alkynyl" or "substituted cycloalkyl" refers to an alkyl, alkenyl, alkynyl or cycloalkyl group substituted with one or more non-interfering substituents, such as, but not limited to, C3-C8 cycloalkyl, e.g., cyclopropyl, cyclobutyl, and the like; acetylene; cyano; alkoxy, e.g., methoxy, ethoxy, and the like; lower alkanoyloxy, e.g., acetoxy; hydroxy; carboxyl; amino; lower alkylamino, e.g., methylamino; ketone; halo, e.g. chloro or bromo; phenyl; substituted phenyl, and the like.

"Alkoxy" refers to an -O-R group, wherein R is alkyl or substituted alkyl, preferably C1-C6 alkyl (e.g., methoxy or ethoxy).

"Aryl" means one or more aromatic rings, each of 5 or 6 core carbon atoms. Multiple aryl rings may be fused, as in naphthyl or unfused, as in biphenyl. Aryl rings may also be fused or unfused with one or more cyclic hydrocarbon, heteroaryl, or heterocyclic rings.

"Substituted aryl" is aryl having one or more non-interfering groups as substituents. For substitutions on a phenyl ring, the substituents may be in any orientation (i.e., ortho, meta or para).

"Heteroaryl" is an aryl group containing from one to four heteroatoms, preferably N, O, or S, or a combination thereof, which heteroaryl group is optionally substituted at carbon or nitrogen atom(s) with C1-6 alkyl, -CF₃, phenyl, benzyl, or thienyl, or a carbon atom in the heteroaryl group together with an oxygen atom form a carbonyl group, or which heteroaryl group is optionally fused with a phenyl ring. Heteroaryl rings may also be fused with one or more cyclic hydrocarbon, heterocyclic, aryl, or heteroaryl rings. Heteroaryl includes, but is not limited to, 5-membered heteroaryls having one hetero atom (e.g., thiophenes, pyrroles, furans); 5-membered heteroaryls having two heteroatoms in 1,2 or 1,3 positions (e.g., oxazoles, pyrazoles, imidazoles, thiazoles, purines); 5-membered heteroaryls having three heteroatoms (e.g., triazoles, thiadiazoles); 5-membered heteroaryls having 3 heteroatoms; 6-membered heteroaryls with one heteroatom (e.g., pyridine, quinoline, isoquinoline, phenanthrine, 5,6-cycloheptenopyridine); 6-membered heteroaryls with two heteroatoms (e.g., pyridazines, cinnolines, phthalazines, pyrazines, pyrimidines, quinazolines); 6-membered heteroaryls with three heteroatoms (e.g., 1,3,5-triazine); and 6-membered heteroaryls with four heteroatoms.

"Substituted heteroaryl" is heteroaryl having one or more non-interfering groups as substituents.

"Heterocycle" or "heterocyclic" means one or more rings of 5-12 atoms, preferably 5-7 atoms, with or without unsaturation or aromatic character and at least one ring atom which is not carbon. Preferred heteroatoms include sulfur, oxygen, and nitrogen. Multiple rings may be fused, as in quinoline or benzofuran.

"Substituted heterocycle" is heterocycle having one or more side chains formed from non-interfering substituents.

"Non-interfering substituents are those groups that, when present in a molecule, are typically non-reactive with other functional groups contained within the molecule.

Suitable non-interfering substituents or radicals include, but are not limited to, halo, C1-C10 alkyl, C2-C10 alkenyl, C2-C10 alkynyl, C1-C10 alkoxy, C7-C12 aralkyl, C7-C12 alkaryl, C3-C10 cycloalkyl, C3-C10 cycloalkenyl, phenyl, substituted phenyl, toluoyl, xylenyl, biphenyl, C2-C12 alkoxyalkyl, C7-C12 alkoxyaryl, C7-C12 aryloxyalkyl, C6-C12 oxyaryl, C1-C6 alkylsulfinyl, C1-C10 alkylsulfonyl, -(CH₂)ₘ-O-(C1-C10 alkyl) wherein m is from 1 to 8, aryl, substituted aryl, substituted alkoxy, fluoroalkyl, heterocyclic radical, substituted heterocyclic radical, nitroalkyl, -NO₂, -CN, -NRC(O)-(C1-C10 alkyl), -C(O)-(C1-C10 alkyl), C2-C10 thioalkyl, -C(O)O-(C1-C10 alkyl), -OH, -SO₂, =S, -COOH, -NR, carbonyl,-C(O)-(C1-C10 alkyl)-CF₃, -C(O)-CF₃, -C(O)NR₂, -(C1-C10 alkyl)-S-(C6-C12 aryl), -C(O)-(C6-C12 aryl), -(CH₂)ₘ-O-(CH₂)ₘ-O-(C1-C10 alkyl) wherein each m is from 1 to 8, -C(O)NR, -C(S)NR, -SO₂NR, -NRC(O)NR, -NRC(S)NR, salts thereof, and the like. Each R as used herein is H, alkyl or substituted alkyl, aryl or substituted aryl, aralkyl, or alkaryl.

"Heteroatom" means any non-carbon atom in a hydrocarbon analog compound. Examples include oxygen, sulfur, nitrogen, phosphorus, arsenic, silicon, selenium, tellurium, tin, and boron.

As used herein, "non-peptidic" refers to a polymer backbone substantially free of peptide linkages. However, the polymer backbone may include a minor number of peptide linkages spaced along the length of the backbone, such as, for example, no more than about 1 peptide linkage per about 50 monomer units.

"Polypeptide" refers to any molecule comprising a series of amino acid residues, typically at least about 10-20 residues, linked through amide linkages (also referred to as peptide linkages) along the alpha carbon backbone. While in some cases the terms may be used synonymously herein, a polypeptide is a peptide typically having a molecular weight up to about 10,000 Da, while peptides having a molecular weight above that are commonly referred to as proteins. Modifications of the peptide side chains may be present, along with glycosylations, hydroxylations, and the like. Additionally, other non-peptidic molecules, including lipids and small drug molecules, may be attached to the polypeptide.

"Amino acid" refers to any compound containing both an amino group and a carboxylic acid group. Although the amino group most commonly occurs at the position adjacent to the carboxy function, the amino group may be positioned at any location within the molecule. The amino acid may also contain additional functional groups, such as amino, thio, carboxyl, carboxamide, imidazole, etc. An amino acid may be synthetic or naturally occurring, and may be used in either its racemic or optically active (D-, or L-) forms, including various ratios of enantiomers.

"Oligomer" refers to short monomer chains comprising 2 to about 10 monomer units, preferably 2 to about 5 monomer units.

The term "conjugate" is intended to refer to the entity formed as a result of covalent attachment of a molecule, e.g., a retinoid, to a reactive polymer molecule, preferably a poly(ethylene glycol).

"Bifunctional" in the context of a polymer of the invention refers to a polymer possessing two reactive functional groups which may be the same or different.

"Multifunctional" in the context of a polymer of the invention means a polymer having 3 or more functional groups attached thereto, where the functional groups may be the same or different. Multifunctional polymers of the invention will typically comprise from about 3-100 functional groups, or from 3-50 functional groups, or from 3-25 functional groups, or from 3-15 functional groups, or from 3 to 10 functional groups, or will contain 3, 4, 5, 6, 7, 8, 9 or 10 functional groups attached to the polymer backbone.

"Dry powder" refers to a powder composition that typically contains less than about 10% moisture.

A composition that is "suitable for pulmonary delivery" refers to a composition that is capable of being aerosolized and inhaled by a subject so that a portion of the aerosolized particles reach the lungs to permit penetration into the lower respiratory tract and alveoli. Such a composition is considered to be "respirable" or "inhaleable".

"Aerosolized" particles are liquid or solid particles that are suspended in a gas, typically as a result of actuation (or firing) of an inhalation device such as a dry powder inhaler, an atomizer, a metered dose inhaler, or a nebulizer.

"Emitted Dose" or "ED" provides an indication of the delivery of a drug formulation from a suitable inhaler device after a firing or dispersion event. More specifically, for dry powder formulations, the ED is a measure of the percentage of powder which is drawn out of a unit dose package and which exits the mouthpiece of an inhaler device. The ED is defined as the ratio of the dose delivered by an inhaler device to the nominal dose (*i.e*., the mass of powder per unit dose placed into a suitable inhaler device prior to firing). The ED is an experimentally-determined parameter, and is typically determined using an *in-vitro* device set up which mimics patient dosing. To determine an ED value, a nominal dose of dry powder, typically in unit dose form, is placed into a suitable dry powder inhaler (such as that described in U.S. Patent No. 5,785,049, assigned to Inhale Therapeutic Systems) which is then actuated, dispersing the powder. The resulting aerosol cloud is then drawn by vacuum from the device, where it is captured on a tared filter attached to the device mouthpiece. The amount of powder that reaches the filter constitutes the emitted dose. For example, for a 5 mg dry powder-containing dosage form placed into an inhalation device, if dispersion of the powder results in the recovery of 4 mg of powder on a tared filter as described above, then the emitted dose for the dry powder composition is: 4 mg (delivered dose)/5 mg (nominal dose) x 100 = 80%. For non-homogenous powders, ED values provide an indication of the delivery of drug from an inhaler device after firing rather than of dry powder, and are based on amount of drug rather than on total powder weight. Similarly for MDI and nebulizer dosage forms, the ED corresponds to the percentage of drug which is drawn from a dosage form and which exits the mouthpiece of an inhaler device.

"Fine particle dose" or "FPD" is defined as the mass percent of powder particles having an aerodynamic diameter less than 3.3 µm, typically determined by measurement in an Andersen cascade impactor.

A "dispersible" or "dispersive" powder is one having an ED value of at least about 30%, more preferably 40-50%, and even more preferably at least about 50-60% or greater.

"Mass median diameter" or "MMD" is a measure of mean particle size, since the powders of the invention are generally polydisperse (*i.e.,* consist of a range of particle sizes). MMD values as reported herein are determined by centrifugal sedimentation, although any number of commonly employed techniques can be used for measuring mean particle size (e.g., electron microscopy, light scattering, laser diffraction).

"Mass median aerodynamic diameter" or "MMAD" is a measure of the aerodynamic size of a dispersed particle. The aerodynamic diameter is used to describe an aerosolized powder in terms of its settling behavior, and is the diameter of a unit density sphere having the same settling velocity, in air, as the particle. The aerodynamic diameter encompasses particle shape, density and physical size of a particle. As used herein, MMAD refers to the midpoint or median of the aerodynamic particle size distribution of an aerosolized powder determined by cascade impaction, unless otherwise indicated.

"Pharmaceutically acceptable excipient or carrier" refers to an excipient that may optionally be included in the compositions of the invention. Preferred for compositions for inhalation are excipients that can be taken into the lungs with no significant adverse toxicological effects to the subject, and particularly to the lungs of the subject.

"Pharmacologically effective amount" or "physiologically effective amount" is the amount of a polymer-retinoic acid conjugate present in a therapeutic composition as described herein that is needed to provide an efficacious level of retinoic acid for treatment of a target condition responsive to treatment with the retinoid. The precise amount will depend upon numerous factors, e.g., the particular PEG-retinoid, the delivery device employed, the components and physical characteristics of the therapeutic composition, intended patient population, patient considerations, and the like, and can readily be determined by one skilled in the art, based upon the information provided herein.

### II. Retinoids

Retinoids for use in the present invention include the natural retinoids as well as synthetic analogs and pharmaceutically acceptable salts and esters thereof. Representative retinoids for use in preparing a polymer conjugate in accordance with the invention include retinol, all trans retinoic acid, 13-cis retinoic acid, 9-cis retinoic acid, 11-cis retinoic acid, and 14-hydroxy-retro-retinol. Preferred are the lipophilic retinoic acid compounds, all trans retinoic acid (ATRA), 13-cis retinoic acid, 9-cis retinoic acid, 11-cis retinoic acid. For ease of reference, the structures of the unmodified parent cis and trans retinoic acids are provided below:

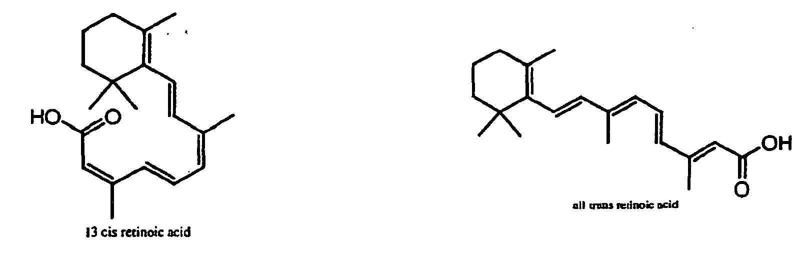

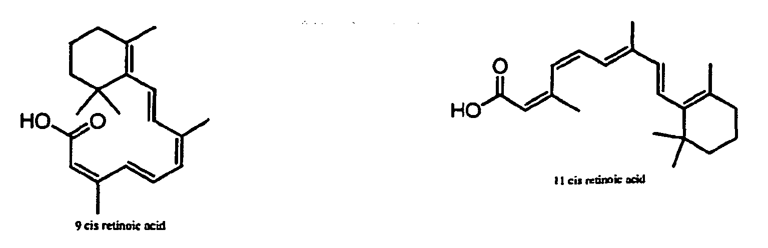

Covalent attachment of the polymer chain can occur at any position within the retinoid moiety that is suitable for chemical modification, although most preferably attachment of a polymer will occur by reaction of a suitably activated polymer with the carbonyl carbon of the carboxylic acid moiety.

Particularly preferred retinoic acids are those that have been shown to be effective in the treatment of certain respiratory disorders such as emphysema, chronic bronchitis, and asthma, e.g., all trans retinoic acid (Massaro G., Massaro, D., *Nature Medicine,* 3:675-677, 1997) and 13-cis retinoic acid (Belloni, P., U.S. Patent No. 6,339,107).

### III. The Polymer

In general, the water soluble and non-peptidic polymer portion of the conjugate is non-toxic and biocompatible, and is typically characterized as having from 2 to about 300 termini. Examples of such polymers include, but are not limited to, poly(alkylene glycols) such as polyethylene glycol (PEG), poly(propylene glycol) ("PPG"), copolymers of ethylene glycol and propylene glycol and the like, poly(oxyethylated polyol), poly(olefinic alcohol), poly(vinylpyrrolidone), poly(hydroxyalkylmethacrylamide), poly(hydroxyalkylmethacrylate), poly(α-hydroxy acid), poly(vinyl alcohol), polyphosphazene, polyoxazoline, poly(N-acryloylmorpholine), and copolymers, terpolymers, and mixtures thereof.

One particularly preferred polymer is polyethylene glycol. The term PEG as used herein includes poly(ethylene glycol) in any of a number of geometries or forms, including its linear forms (e.g., alkoxy PEG or bifunctional PEG), branched or multi-arm forms (e.g., forked PEG or PEG attached to a polyol core), pendant PEG, or PEG with degradable linkages therein, to be more fully described below.

Typically, PEG is activated with a suitable activating group appropriate for coupling to a desired site on the retinoid such as the carbonyl carbon. An activated PEG will possess a reactive group at a terminus for reaction with a retinoid. The term "linker" as used herein is meant to encompass an activating group positioned at a PEG terminus for reaction with a retinoid such as retinoic acid, and may further include additional (typically inert) atoms positioned between the PEG portion of the polymer and the activated group at the terminus, for ease in preparing the activated PEG. The linkers may contain any of a number of atoms, however, preferred are linkers containing methylenes intervening between the PEG backbone and the terminal activating group. Representative activated PEG derivatives and methods for conjugating these polymers to a drug such as a retinoid are known in the art and further described in Zalipsky, S., et al., *"Use of Functionalized Poly(Ethylene Glycols) for Modification of Polypeptides"* in Polyethylene Glycol Chemistry: Biotechnical and Biomedical Applications, J. M. Harris, Plenum Press, New York (1992), and in Advanced Drug Reviews, **16**:157-182 (1995).

Typically, the number average molecular weight of the polymer portion of a polymer conjugate of the invention is from about 100 daltons (Da) to about 100,000 Da, preferably about 500 daltons to about 100,000 daltons.

More specifically, a PEG-retinoid conjugate of the invention will typically comprise one or more PEG chains each having a molecular weight ranging from about 200 to about 40,000 daltons, and preferably ranging from about 200 to about 20,000 daltons. Preferably, a PEG for use in the invention will possess an average molecular weight falling within one of the following ranges: from about 200 to 10,000 daltons, from about 200 to about 7500 daltons, from about 200 to about 6,000 daltons, from about 200 to about 5,000 daltons, from about 200 to about 3000 daltons, from about 200 to about 2000 daltons, and from about 200 to about 1000 daltons.

Polymers for attachment to a retinoid will generally possess a number average molecular weight selected from the following: (i) about 500 Da, or (ii) about 750 Da, or (iii) about 900 Da, or (iv) about 1,000 Da, or (v) about 2,000 Da, or (vi) about 3,000 Da, or (vii) about 4,000 Da, or (viii) about 5.000 Da, or (ix) about 10,000 Da, or (x) about 15,000 Da, or (xi) about 20,000, or (xii) about 25,000 Da. In one particular embodiment of the invention, preferred are polymers having a molecular weight of greater than about 2000 daltons. Exemplary retinoid conjugates prepared with PEGs having molecular weights of 5,000 daltons, 20,00 daltons, 2000 daltons, and 10,000 daltons (using both linear and multi-armed PEGs) are described in the Examples.

In terms of the number of subunits, PEGs for use in the invention will typically comprise a number of (OCH₂CH₂) subunits falling within one or more of the following ranges: 2 to about 900 subunits, from about 4 to about 450 subunits, from about 4 to about 250 subunits, from about 4 to about 170 subunits, from about 4 to about 140 subunits, from about 4 to about 100 subunits, from about 10 to about 100 subunits, from about 4 to about 70 subunits, from about 4 to about 45 subunits, and from about 4 to about 25 subunits.

One particularly preferred polymer for use in the invention is an end-capped polymer, meaning a polymer having at least one terminus capped with a relatively inert group, such as a lower C1-C6 alkoxy group. One such particularly preferred form of PEG is methoxy-PEG (commonly referred to as mPEG), a linear form of PEG wherein one terminus of the polymer is a methoxy (-OMe) group, while the other terminus is a hydroxyl or other functional group that can be chemically modified.

Multi-armed or branched PEG molecules, such as those described in U.S. Patent No. 5,932,462, can also be used to form a conjugate of the invention. Generally speaking, a multi-armed or branched polymer possesses two or more polymer "arms" extending from a central branch point (e.g., C in the structure below) that is covalently attached, either directly or indirectly via intervening connecting atoms, to one active moiety, such as a retinoid. For example, an exemplary branched PEG polymer can have the structure: 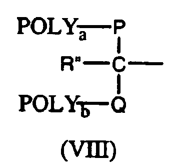 wherein:
POLYₐ and POLY_{b} are PEG polymers, such as methoxy poly(ethylene glycol);
R" is a nonreactive moiety, such as H, methyl or PEG; and
P and Q are nonreactive linkages. In a preferred embodiment, the branched PEG polymer is methoxy poly(ethylene glycol) disubstituted lysine or a derivative thereof. Depending upon the active moiety within the retinoid intended for coupling to the polymer, the reactive ester function of the disubstituted lysine may be further modified to form a functional group suitable for reaction with the target group within the retinoid.

The polymer may alternatively have a forked structure. Generally speaking, a polymer having a forked structure is characterized as having a polymer chain attached to two or more active agents via covalent linkages extending from a hydrolytically stable branch point in the polymer. An example of a forked PEG is represented by PEG-YCHZ_{2,} where Y is a linking group and Z is an activated terminal group for covalent attachment to a biologically active agent, such as a retinoid. The Z group is linked to CH by a chain of atoms of defined length. International Application No. PCT/US99/05333, describes various forked PEG structures suitable for use in the present invention. The chain of atoms linking the Z functional groups to the branching carbon atom serve as a tethering group and may comprise, for example, an alkyl chain, ether linkage, ester linkage, amide linkage, or combinations thereof.

A PEG polymer may also take the form of a pendant PEG molecule having reactive groups, such as hydroxyl, covalently attached along the length of the PEG backbone rather than at the ends of the PEG chain. Such pendant reactive groups may be attached to the PEG backbone directly or through a linking moiety, such as an alkylene group.

In addition to the above-described forms of PEG, the polymer can also be prepared with one or more weak or degradable linkages in the polymer backbone, including any of the above described polymers. That is to say, in addition to the linkage coupling the polymer to the retinoid, the polymer may contain additional hydrolyzable bonds within the polymer to provide further degradation of the polymer, e.g., upon deposition of the conjugate in the lung of a subject subsequent to administration by inhalation. For example, a PEG can be prepared with ester linkages in the polymer backbone that are subject to hydrolysis. As shown below, this hydrolysis results in cleavage of the polymer into fragments of lower molecular weight:

-PEG-CO₂-PEG- + H₂O → -PEG-CO₂H + HO-PEG-

Other hydrolytically degradable linkages that may be contained within the polymer backbone include carbonate linkages; imine linkages resulting, for example, from reaction of an amine and an aldehyde (see, e.g., Ouchi et al., Polymer Preprints, 38(1):582-3 (1997); phosphate ester linkages formed, for example, by reacting an alcohol with a phosphate group; hydrazone linkages which are typically formed by reaction of a hydrazide and an aldehyde; acetal linkages that are typically formed by reaction between an aldehyde and an alcohol; ortho ester linkages that are, for example, formed by reaction between a formate and an alcohol; peptide linkages formed by an amine group, e.g., at an end of a polymer such as PEG, and a carboxyl group of a peptide; and oligonucleotide linkages formed by, for example, a phosphoramidite group, e.g., at the end of a polymer, and a 5' hydroxyl group of an oligonucleotide.

Such optional features of the polymer conjugate, i.e., the introduction of one or more degradable linkages into the polymer chain, may provide for additional control over the final desired pharmacological properties of the conjugate upon administration. For example, a large and relatively inert conjugate (i.e., having one or more high molecular weight PEG chains attached thereto, e.g., one or more PEG chains having a molecular weight greater than about 10,000, wherein the conjugate possesses essentially no bioactivity) may be administered, which when in the lung, is hydrolyzed to generate a bioactive conjugate possessing a portion of the original PEG chain. In this way, the properties of the PEG-retinoid conjugate may be somewhat more effectively tailored to balance the bioactivity of the conjugate and the depot effect (residence time) of the retinoid within the lung.

In sum, any of a variety of monofunctional, bifunctional or multifunctional polymers that are non-peptidic and water-soluble as defined in Claim 1 can be used to form a retinoid conjugate in accordance with the present invention. The polymer can be linear, or can be in any of the above-described forms (e.g., branched, forked, and the like).

### IV. Polymer Retinoid Conjugates

As described generally above, a polymer conjugate of the invention comprises a water-soluble and non-peptidic polymer covalently attached to a retinoid compound, where the polymer portion of the conjugate may possess any of the exemplary forms described in section III above. In instances where the retinoid is a retinoic acid, preferred conjugates are those where the linkage connecting the retinoic acid to the polymer is a derivative of the parent carboxylic acid group, such as an ester, an amide or a thiol ester. The polymer conjugates of the invention are useful for the treatment of any condition responsive to retinoid therapy and are particularly beneficial for use in chemoprevention, particularly of aerodigestive cancers, such as lung cancer as well as carcinomas and malignancies of the head and neck. Moreover, localized administration of the polymer conjugates of the invention by inhalation results in a higher fraction of the dose reaching the desired site of action, i.e, the alveolar regions, when compared to systemic administration. In this manner, the polymer conjugates of the invention are capable of minimizing the common adverse side effects of systemic retinoid therapy caused by toxic levels of retinoids in the circulation. Moreover, due to the aqueous solubility of the conjugates of the invention, aqueous based delivery vehicles and aqueous processing conditions during processes such as spray drying can be employed without having to resort to solubilizing agents or the hazards of spray drying with organic solvents.

The conjugates of the invention are preferably although not necessarily prodrugs, meaning that the linkage between the polymer and the retinoid is hydrolytically degradable to allow release of the parent retinoid. Exemplary degradable linkages include carboxylate ester, phosphate ester, thiolester, anhydrides, acetals, ketals, acyloxyalkyl ether, imines, orthoesters, peptides and oligonucleotides. Such linkages can be readily prepared by appropriate modification of either the retinoid moiety (e.g., the carboxyl group on a retinoid or a hydroxy group on a retinol) and/or the polymer using coupling methods commonly employed in the art. However, most preferred are hydrolyzable linkages that are readily formed by reaction of a suitably activated polymer with a non-modified functional group contained within the parent molecule. For instance, most preferred for modification of a retinoic acid is an ester or thiolester linkage resulting from attack on the carbonyl carbon of the retinoid by either a hydroxy or thiol group on the polymer.

Alternatively, a hydrolytically stable linkage, such as an amide, urethane (also known as carbamate), amine, thioether (also known as sulfide), or urea (also known as carbamide) linkage can also be employed for coupling the retinoid to the polymer. Again, a preferred hydrolytically stable linkage is an amide, due to the straightforward nature of the chemistry. That is to say, an amide may be readily prepared by reaction of a retinoic acid (or a functional equivalent thereof) with an amino-terminated polymer. The particular linkage and linkage chemistry employed will depend upon the particular retinoid molecule, functional groups within the retinoid available either for attachment to a polymer or conversion to a suitable attachment site, the possible presence of additional functional groups within the retinoid molecule, and the like, and can be readily determined by one skilled in the art based upon the guidance presented herein.

The polymer conjugates of the invention may or may not possess a measurable degree of retinoic activity. That is to say, a polymer conjugate in accordance with the invention will possesses anywhere from about 0% to about 100% or more of the bioactivity of the unmodified parent retinoid compound. Compounds possessing little or no retinoic bioactivity will typically contain a hydrolyzable linkage connecting the polymer to the retinoid moiety, so that regardless of the lack of bioactivity in the water-soluble prodrug, the active parent molecule is released upon aqueous-induced cleavage of the hydrolyzable linkage. Such activity may be determined using a suitable *in-vivo* or *in-vitro* model, depending upon the known activity of the particular retinoid parent compound employed. For conjugates possessing a hydrolytically stable linkage coupling the retinoid to the polymer, the conjugate will typically possess a measurable degree of specific activity. For instance, such polymer conjugates are typically characterized as having a bioactivity of at least about 2%, 5%, 10%, 15%, 25%, 30%, 40%, 50%, 60%, 80%, 90% or more relative to that of the unmodified parent retinoid, when measured in a suitable model, such as those well known in the art. Preferably, compounds having a hydrolytically stable linkage (e.g., an amide linkage) will possess at least about 40% of the bioactivity of the unmodified parent retinoid.

Exemplary polymer conjugates in accordance with the invention will now be described. A representative polymer conjugate in accordance with the invention may generally be characterized by the following structure, 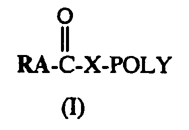 where RA together with the adjacent carbonyl group represents a retinoid moiety, X is a linker, preferably a heteroatom selected from O, N-H, and S, and POLY is a water soluble, non-peptidic polymer.

In one instance, for example, where the polymer is a linear polyethylene glycol and the linkage connecting the retinoid to the polymer is an ester linkage (e.g., X is an O), the molecular weight of the polymer will preferably be at least about 2,000 daltons. Alternatively, on occasions where the polymer is a linear end-capped polyethylene glycol, and the linkage is an amide linkage (e.g., X is a N-H), then the polymer will preferably have a molecular weight of at least about 5,000 daltons.

The C(O)-X linkage results from the reaction of a functional group at a terminus of the polymer with a retinoic acid molecule. As discussed above, the specific linkage will depend on the type of functional group utilized. If the polymer is end-functionalized or "activated" with a hydroxyl group, the resulting linkage will be a carboxylic acid ester and X will be O. If the polymer backbone is functionalized with a thiol group, the resulting linkage will be a thioester and X will be S. When certain multi-arm, branched or forked polymers are employed, the C(O)X moiety, and in particular the X moiety, may be relatively more complex and may include a longer linkage structure. For example, as shown below in the "forked" polymer embodiment, the X portion of the conjugate is a linker corresponding to a -X₁(W)ₚ-CH-Y₁- linkage between the polymer and the retinoic acid moiety.

Particularly preferred conjugates will have the generalized structures presented below where X and POLY are as described above.

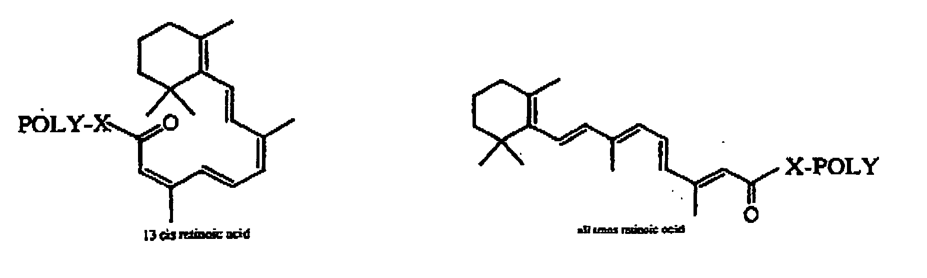

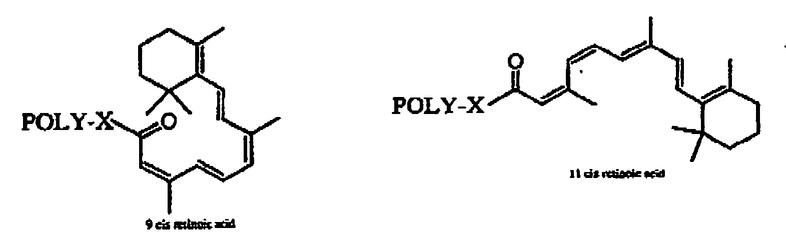

Alternatively, a conjugate of the invention may possess a structure of the following type, 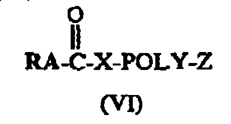 where the one terminus of the modifying polymer is end-functionalized with a functional group, Z. The functional group Z may be an end-capping group such as alkoxy or benzyloxy, or may be a reactive functional group such as hydroxyl, active ester, active carbonate, acetal, aldehyde, aldehyde hydrate, alkenyl, acrylate, methacrylate, acrylamide, active sulfone, amine, hydrazide, thiol, carboxylic acid, isocyanate, isothiocyanate, maleimide, vinylsulfone, dithiopyridine, vinylpyridine, iodoacetamide, epoxide, glyoxal, dione, mesylate, tosylate, tresylate, or functional equi valents thereof.

Specific examples of suitable terminal functional groups include N-succinimidyl carbonate (see e.g., U.S. Patent Nos. 5,281,698, 5,468,478), amine (see, e.g., Buckmann et al. Makromol. Chem. 182:1379 (1981), Zaplipsky et al. Eur. Polym. J. 19:1177 (1983)), hydrazide (See, e.g., Andresz et al. Makromol. Chem. 179:301 (1978)), succinimidyl propionate and succinimidyl butanoate (see, e.g., Olson et al. in Poly(ethylene glycol) Chemistry & Biological Applications, pp 170-181, Harris & Zaplipsky Eds., ACS, Washington, DC, 1997; see also U.S. Patent No. 5,672,662), succinimidyl succinate (See, e.g., Abuchowski et al. Cancer Biochem. Biophys. 7:175 (1984) and Joppich et al. Macrolol. Chem. 180:1381 (1979), succinimidyl ester (see, e.g., U.S. Patent No. 4,670,417), benzotriazole carbonate (see, e.g., U.S. Patent No. 5,650,234), glycidyl ether (see, e.g., Pitha et al. Eur. J. Biochem. 94:11 (1979), Elling et al., Biotech. Appl. Biochem. 13:354 (1991), oxycarbonylimidazole (see, e.g., Beauchamp, et al., Anal. Biochem. 131:25 (1983), Tondelli et al. J. Controlled Release 1:251 (1985)), p-nitrophenyl carbonate (see, e.g., Veronese, et al., Appl. Biochem. Biotech., 11:141 (1985); and Sartore et al., Appl. Biochem. Biotech., 27:45 (1991)), aldehyde (see, e.g., Harris et al. J. Polym. Sci. Chem. Ed. 22:341 (1984), U.S. Patent No. 5,824,784, U.S. Patent 5,252,714), maleimide (see, e.g., Goodson et al. Bio/Technology 8:343 (1990), Romani et al. in Chemistry of Peptides and Proteins 2:29 (1984)), and Kogan, Synthetic Comm. 22:2417 (1992)), orthopyridyl-disulfide (see, e.g., Woghiren, et al. Bioconj. Chem. 4:314 (1993)), acrylol (see, e.g., Sawhney et al., Macromolecules, 26:581 (1993)), vinylsulfone (see, e.g., U.S. Patent No. 5,900,461).

In one embodiment, a conjugate characterized as having the generalized structure of (VI) above is one where Z corresponds to 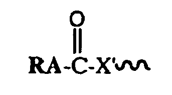 where X' together with the carbonyl is a hydrolytically degradable linkage and 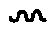 represents the point of attachment to the polymer. Such conjugates are referred to herein as having a dumbell structure where a central and typically linear polymer possesses a retinoid covalently attached at each end. The particular embodiment described immediately above is considered a homobifunctional polymer conjugate when X and X' are identical, since both ends of the polymer are modified to possess functionalities that are the same (hence the term, "homo").

Also provided herein are conjugates having a multi-armed or branched structure such as the following: 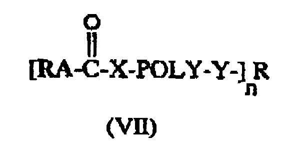 The central core molecule is derived from a molecule having n number of functional sites capable of attaching to n number of polymers, POLY, via a linkage, Y. Typically, such conjugates will possess n values ranging from about 3 to 100 or more, typically from about 3 to about 20 or even from about 3 to about 10. R designates a central core molecule that is preferably non-dendritic such as a polyol, a polyamine, or a molecule having a combination of amino and hydroxyl groups. Examples of specific core molecules include glycerol, glycerol oligomers, pentaerythritol, sorbitol, and lysine. RA, X and POLY are as defined above (where each POLY is independently selected) and Y represents a linker. Preferably, the molecular weight of R is less than about 2,000 Da.

One such particularly preferred conjugate possesses the following, structure: 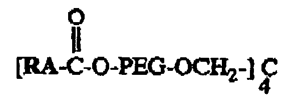 where PEG designates a polyethylene glycol having a MW from about 2,000 Da to about 100,000 Da, or one of the alterative PEG molecular weight ranges described above.
In an embodiment of the invention where the retinoid is ATRA, the conjugate will possess the stucture shown below.

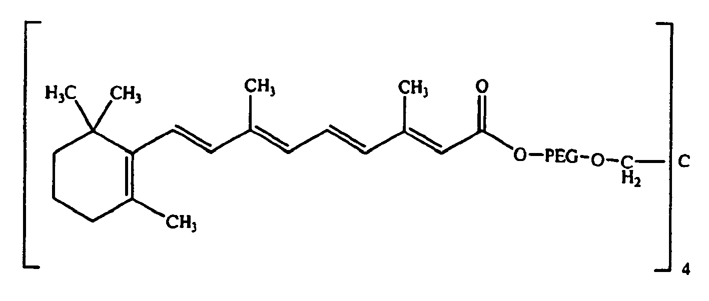

A specific example of an illustrative "forked" polymer conjugate of the invention is shown below: 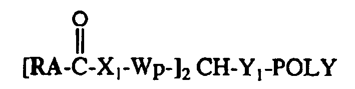 wherein each X₁ together with the carbonyl forms a hydrolytically degradable linkage, e.g., X₁ is an atom or group of atoms such as O or S, Y₁ is a linker, such as O, S or NH, each p is independently 0 or 1, and each W is independently selected from the group consisting of -(CH₂)ₘ -, -(CH₂)ₘ-O-, -O-(CH₂)ₘ-, -(CH₂)ₘ-O₂C-CH₂CH₂-, and -(CH₂)ₘ-O-(CH₂)ᵣ-, wherein m and r are independently 1-10. Preferably, each p is 1 and each W is -(CH₂)ₘ -, wherein each m is independently 1-10.

In one embodiment of the above forked polymer conjugate, Y₁ is bonded to - POLY-Z, wherein POLY is a water soluble and non-peptidic polymer and Z is a functional group such as an inert capping group as described above, e.g., 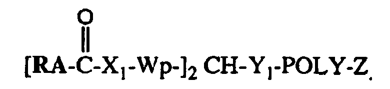 Alternatively, Z may be a reactive functional group such as hydroxyl, active ester, active carbonate, acetal, aldehyde, aldehyde hydrate, alkenyl, acrylate, methacrylate, acrylamide, active sulfone, amine, hydrazide, thiol, carboxylic acid, isocyanate, isothiocyanate, maleimide, vinylsulfone, dithiopyridine, vinylpyridine, iodoacetamide, epoxide, glyoxal, dione, mesylate, tosylate, tresylate or functional equivalents thereof.

An illustrative homobifunctional forked polymer conjugate is one having the generalized structure shown above where Z is

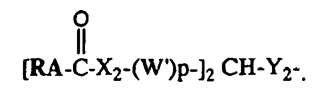

### V. Methods of Preparation

A conjugate as described herein is prepared using coupling methods commonly employed in the art. A polymer retinoid conjugate may by formed, e.g., by reacting a water soluble and non-peptidic polymer activated with a functional group, e.g., a hydroxyl or thiol, that reacts with a carboxylic acid group or an activated carboxylic acid such as an acid halide. In such instances, the resulting polymer conjugate is a prodrug of retinoic acid having, for example, a carboxylic acid ester or thioester linkage between the polymer and the retinoic acid moiety. As described in the accompanying examples, for coupling reactions carried out with retinoic acid, the reaction preferably occurs in the presence of a catalytic amount of a coupling reagent such as N,N'-dicyclohexylcarbodiimide (DCC) or N,N'-dimethylaminopyridine (DMAP). Suitable reaction conditions for preparing an ester-linked conjugate by reaction of a hydroxy-terminated polymer with the carboxy group of retinoic acid can be readily determined by one skilled in the art, e.g., using any of a number of routes such as those described in *"Comprehensive Organic Transformations",* Larock R., VCH Publishers, 1989. Alternatively, in reactions conducted with an acid halide form of retinoic acid, the acid halide may be formed by reacting retinoic acid with an oxalyl halide, such as oxalyl chloride, which is then coupled to the polymer as described generally above. Preparation of the corresponding amide or thioester linked conjugates may also be prepared using conventional synthetic methodologies.

The final polymer conjugate product is generally purified and collected by precipitation followed by filtration and drying.

An example of a suitable reaction scheme for preparing a conjugate of the invention is shown below, wherein Z is a capping group, POLY is a water soluble and non-peptidic polymer, and X is O or S:

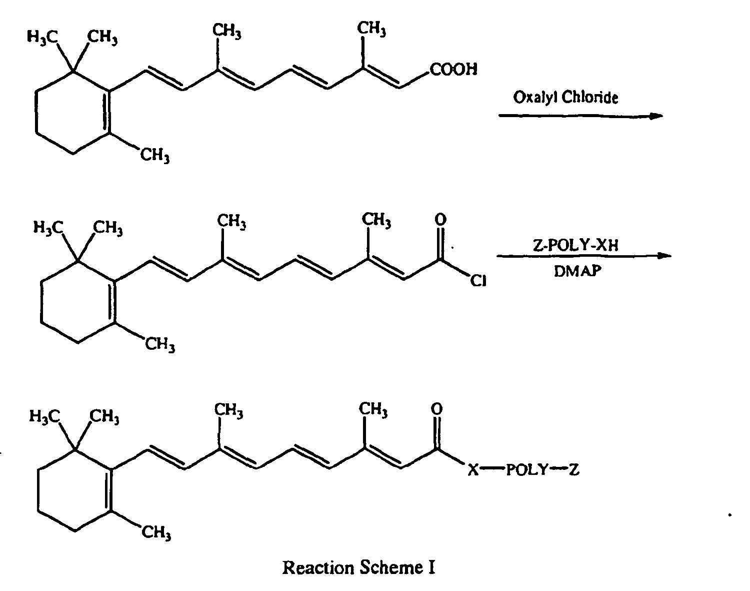

### VI. Compositions

The polymer-retinoid conjugate compositions of the invention may be administered neat or in therapeutic/pharmaceutical compositions containing additional excipients, solvents, stabilizers, etc., depending upon the particular mode of admistration and dosage form. Specific administration routes include oral, nasal, and pulmonary. Most preferred is the pulmonary route, particularly for treatment of diseases of the respiratory tract, especially those associated with tobacco use or exposure.

Pharmaceutical formulations for mammalian and preferably human administration will typically comprise at least one PEG-retinoid conjugate of the invention together with one or more pharmaceutically acceptable carriers, as will be described in greater detail below, particularly for pulmonary compositions. Formulations of the present invention are most typically liquid solutions or suspensions, while inhaleable formulations for pulmonary administration are generally liquids or powders, with powder formulations being generally preferred. Additional albeit less preferred, compositions of the chemically modified retinoids of the invention include syrups, creams, ointments, tablets, and the like.

### A. Inhaleable Formulations of Polymer Retinoid Conjugates

As stated above, the route of administration for the conjugates of the invention is by inhalation to the lung. Particular formulation components, characteristics and delivery devices will now be more fully described.

The amount of retinoid conjugate in the formulation will be that amount necessary to deliver a therapeutically effective amount of retinoid per unit dose to achieve a desired therapeutic effect. In practice, this will vary widely depending upon the particular retinoid, the polymer portion of the conjugate, its activity, the condition to be treated, etc. The composition will generally contain anywhere from about 1% by weight to about 99% by weight of the polymer conjugate, typically from, about 2% to about 95% by weight conjugate, and more typically from about 5% to 85% by weight conjugate, and will also depend upon the relative amounts of excipients/additives contained in the composition: More specifically, the composition will typically contain at least about one of the following percentages of conjugate: 10%, 20%, 30%, 40%, 50%, 60%, 70%, 80%, 90% or more by weight. Preferably, powder compositions will contain at least about 40%, e.g., from about 40-100% by weight polymer retinoid conjugate. It is to be understood that more than one retinoid may be incorporated into the formulations described herein.

Compositions of the invention will, in most instances, include one or more excipients. For dry powder compositions, preferred are excipients having a high melting point or those having a high glass transition temperature, e.g., above about 35° C, preferably above about 40 °C, more preferably above 45° C, and most preferably above about 55 °C.

Commonly employed are carbohydrate excipients, either alone or in combination with other excipients or additives. Representative carbohydrates for use in the compositions of the invention include sugars, derivatized sugars such as alditols, aldonic acids, esterified sugars, and sugar polymers. Preferred are nonreducing sugars, sugars that can form a partially or substantiate amorphous or glassy phase when combined with a retinoid conjugate, and sugars possessing relatively high Tgs (e.g., Tgs greater than 40°C, preferably greater than 50°C, more preferably greater than 60°C, and even more preferably greater than 70°C, and most preferably having Tgs of 80°C and above).

Additional excipients include amino acids, peptides and particularly oligomers comprising 2-9 amino acids, and more preferably 2-5 mers, and polypeptides, all of which may be homo or hetero species. One particularly preferred amino acid is leucine.

Also useful as an excipient is a di- or tripeptide containing two or more leucyl residues, as described in Inhale Therapeutic System's International patent application PCT/US00/09785. Once such excipient is trileucine. Inhaleable formulations containing trileucine are described in Example 14.

Polyamino acids, and in particular, those comprising any of the herein described amino acids, are also suitable for use as an excipient. Preferred are polyamino acids such as poly-lysine, poly-glutamic acid, and poly(lys, ala).

Additional excipients and additives include but are not limited to proteins, non-biological polymers, and biological polymers, which may be present singly or in combination. Suitable excipients are those provided in Inhale Therapeutic Systems' International Publication Nos. WO 96/32096 and 98/16205. The compositions may also include a buffer or a pH adjusting agent.

Additionally, a composition in accordance with the invention will preferably contain a free radical scavenger such as butylatedhydroxytoluene (BHT), ascorbic acid (vitamin C), or alpha tocopherol (vitamin A). During experiments conducted in support of the present invention, it was discovered that certain PEG-retinoid compositions, e.g., PEG ATRA conjugates such as PEG-5K-ester-ATRA, were more prone to degradation (i.e., were less stable) than compositions of the unmodified parent retinoid, presumably due to the presence of a small amount of free radicals in compositions of PEG-ATRA. Incorporation of a free radical scavenger in the composition was effective to substantially reduce or effectively eliminate free-radical induced degradation of the conjugate.

Advantageously, formulations of the retinoid conjugates of the invention do not require the incorporation of solubilizing or emulsifying agents, due to the water-soluble nature of the conjugates.

The use of certain di-substituted phosphatidylcholines for producing perforated microstructures (i.e., hollow, porous microspheres) is described in greater detail below and described in Example 15. Other pharmaceutical excipients and/or additives suitable for use in the compositions according to the invention are listed in *"Remington: The Science & Practice of Pharmacy",* 19^{th} ed., Williams & Williams, (1995), and in the *"Physician's Desk Reference",* 52^{nd} ed., Medical Economics, Montvale, NJ (1998).

Generally, the pharmaceutical compositions of the invention will contain from about 1% to about 99% by weight excipient, preferably from about 5%-98% by weight excipient, more preferably from about 15-95% by weight excipient. Even more preferably, the composition will contain from about 0-50% by weight excipient, more preferably from 0-40% by weight excipient. In general, a relatively high retinoid concentration (weight percent) is desired in the final pharmaceutical composition. Typically, the optimal amount of excipient/additive is determined experimentally, i.e., by preparing compositions containing varying amounts of excipients (ranging from low to high), examining the chemical and physical stability of the PEG-retinoid, the activity of the conjugate, its absorption from the lung into the circulation, along with the aerosol properties of the composition, and then further exploring the range at which optimal aerosol performance is attained whilst balancing the other factors set forth above.

### B. Preparing Dry Powders

Dry powder formulations of the invention comprising a PEG-retinoid conjugate may be prepared by any of a number of drying techniques, and preferably by spray drying. Spray drying of the formulations is carried out, for example, as described generally in the "Spray Drying Handbook", 5^{th} ed., K. Masters, John Wiley & Sons, Inc., NY, NY (1991), and in Platz, R., *et al.,* International Patent Publication Nos. WO 97/41833 (1997) and WO 96/32149 (1996).

From a processing standpoint, the conjugate compositions of the invention are preferred over compositions of their unmodified retinoid counterparts for a number of reasons. First, due to their insolubility in water, unmodified retinoids would typically be spray dried either as a suspension or in an organic solvent such as ethanol. Spray drying in an organic solvent can be undesirable due to the generation of large volumes of flammable organic waste streams and the potential for explosion. Moreover, as can be seen in Example 13, using conventional processing parameters, spray drying of a low melting solid such as ATRA results in sublimation of the compound throughout the spray dryer apparatus, and generation of a non-free flowing powder. These problems can be circumvented when spray drying a retinoid conjugate of the invention, since aqueous feed solutions can be employed. Moreover, depending upon the choice of polymer, the polymer portion of the conjugate is not only effective to impart water solubility to the resulting compound, but is also effective in raising the melting point of the conjugate, making spray drying using conventional processing parameters possible. Secondly, due to their low aqueous solubility, water-based formulations cannot be employed for formulating an unmodified retinoid. This is particularly problematic for inhaleable formulations, since the agents required for solubilizing a retinoid typically produce compositions that are unsuitable for aerosol formation. This problem is similarly avoided by the use of a conjugate of the invention.

A solution or suspension of a polymer retinoid conjugate may be spray dried in a conventional spray drier, such as those available from commercial suppliers such as Niro A/S (Denmark), Buchi (Switzerland) and the like, resulting in a dispersible, dry powder as demonstrated in the Examples. Optimal conditions for spray drying the PEG-retinoid solutions will vary depending upon the formulation components, and are generally determined experimentally. The gas used to spray dry the material is typically air, although inert gases such as nitrogen or argon are also suitable. Moreover, the temperature of both the inlet and outlet of the gas used to dry the sprayed material is such that it does not cause degradation or melting of the PEG-retinoid during the spray drying process. Depending upon the melting point of the conjugate, an inlet temperature is selected that results in an outlet temperature that is at least about 10 degrees or preferably 15 degrees lower or more than the melting temperature of the conjugate. Preferably, the melting temperature of the conjugate is first determined to aid in the appropriate selection of outlet temperature and other relevant processing parameters.

Respirable polymer-retinoid compositions having the features described herein may also be produced by drying certain formulation components which result in formation of a perforated microstructure powder as described in WO 99/16419. The perforated microstructure powders typically comprise spray-dried, hollow microspheres having a relatively thin porous wall defining a large internal void. The perforated microstructure powders may be dispersed in a selected suspension media (such as a non-aqueous and/or fluorinated blowing agent) to provide stabilized dispersions prior to drying. The use of relatively low density perforated (or porous) microstructures or microparticulates significantly reduces attractive forces between the particles, thereby lowering the shear forces, increasing the flowability and dispersibility of the resulting powders, and reducing the degradation by flocculation, sedimentation or creaming of the stabilized dispersions thereof.

Alternatively, a PEG-retinoid composition for pulmonary delivery may comprise aerodynamically light particles as described in U.S. Patent No. 6,136,295.

A powdered formulation of the invention may also be prepared by lyophilization, vacuum drying, spray freeze drying, super critical fluid processing (e.g., as described in Hanna, *et al.,* U.S. Patent No. 6,063,138), air drying, or other forms of evaporative drying.

Dry powders may also be prepared by blending, grinding, sieving or jet milling formulation components in dry powder form.

Once formed, a dry powder composition is preferably maintained under dry (*i.e.*, relatively low humidity) conditions with minimal exposure to excessive heat or light during manufacture, processing, and storage.

### C. Features of an Inhaleable Dry Powder Formulation

Powders of the invention are further characterized by several features, most notably, one or more of the following: (i) consistently high dispersibilities, which are maintained, even upon storage (ii) small aerodynamic particles sizes (MMADs), (iii) improved fine particle dose values, i.e., powders having a higher percentage of particles sized less than 3.3 microns MMAD, all of which contribute to the improved ability of the powder to penetrate to the tissues of the lower respiratory tract (*i.e*., the alveoli) for either localized delivery to the lung, or alternatively, when used in the treatment of non-respiratory conditions, absorption into the systemic circulation. These physical characteristics of the inhaleable powders of the invention, to be described more fully below, are important in maximizing the efficiency of aerosolized delivery of such powders to the lung and deep lung.

Dry powders of the invention are composed of aerosolizable particles effective to penetrate into the lungs. The particles of the invention have a mass median diameter (MMD) of less than about 20-30 µm, or less than 20 µm, or less than about 10 µm, preferably less than about 7.5 µm, and more preferably less than about 4 µm, and even less than about 3.5 µm, and usually are in the range of 0.1 µm to 5 µm in diameter. Preferred powders are composed of particles having an MMD from about 0.2 to 4.0 µm. In some cases, the powder will also contain non-respirable carrier particles such as lactose, where the non-respirable particles are typically greater than about 40 µm (microns) in size.

The powders of the invention are further characterized by an aerosol particle size distribution less than about 10 µm mass median aerodynamic diameter (MMAD), preferably having MMADs less than about 5 µm, more preferably less than 4.0 µm, even more preferably less than 3.5 µm, and most preferably less than 3 µm. The mass median aerodynamic diameters of the powders will characteristically range from about 0.1-10 µm, preferably from about 0.2 - 5.0 µm MMAD, more preferably from about 1.0 - 4.0 µm MMAD, and even more preferably from about 1.5 to 3.0 µm. Small aerodynamic diameters can generally be achieved by a combination of optimized spray drying conditions and choice and concentration of excipients.

The powders will generally have a moisture content below about 20% by weight, usually below about 10% by weight, and preferably below about 5% by weight. Preferred powders in accordance with the invention having a moisture content that is below about one or more of the following weight percentages: 15%, 10%, 7%, 5%, or 3%. Such low moisture-containing solids tend to exhibit a greater stability upon packaging and storage.

Additionally, the spray drying methods and stabilizers and excipients described herein are effective to provide highly dispersible polymer conjugate formulations. For powder formulations, the emitted dose (ED) of these powders is typically greater than 30%, and usually greater than 40%. More preferably, the ED of the powders of the invention is greater than 50%, and is often greater than 60%.

### D. Administration of the Conjugate Composition

The polymer-retinoid formulations as described herein may be delivered directly to the lung using any of a number of delivery devices. For example, a dry powder inhaler (DPI), *i.e*., an inhaler device that utilizes the patient's inhaled breath as a vehicle to transport the dry powder drug to the lungs, may be employed. Preferred are Inhale Therapeutic Systems' dry powder inhalation devices as described in Patton, J.S., *et al.,* U.S. Patent No. 5,458,135, Oct. 17, 1995; Smith. A. E., *et al.,* U.S. Patent No. 5,740,794, Apr. 21, 1998; and in Smith, A. E., *et. al.,* U.S. Patent No. 5,785,049. July 28, 1998. When administered using a device of this type, the powdered medicament is contained in a receptacle having a puncturable lid or other access surface, preferably a blister package or cartridge, where the receptacle may contain a single dosage unit or multiple dosage units. Convenient methods for filling large numbers of cavities (i.e., unit dose packages) with metered doses of dry powder medicament are described, e.g., in Parks, D. J., *et al.,* International Patent Publication WO 97/41031, Nov. 6,1997, incorporated herein by reference. Thus, in another aspect, the invention encompasses a unit dosage form of a polymer-retinoid conjugate of the invention for use in an inhaler device.

Other dry powder dispersion devices for pulmonary administration of dry powders include those described, for example, in Newell, R. E., *et al,* European Patent No. EP 129985, Sept. 7. 1988; in Hodson, P. D., *et al.,* European Patent No. EP472598, July 3,1996: in Cocozza, S., *et al.,* European Patent No. EP 467172, April 6. 1994, and in Lloyd, L.J. *et al.,* U.S. Patent No. 5,522,385, June 4, 1996, incorporated herein by reference. Also suitable for delivering PEG-retinoid dry powders are inhalation devices such as the Astra-Draco "TURBUHALER". This type of device is described in detail in Virtanen, R., U.S. Patent No. 4,668,218, May 26, 1987; in Wetterlin, K., *et al.,* U.S. Patent No. 4,667,668, May 26. 1987; and in Wetterlin. K., *et al..* U.S. Patent No. 4.805.811, Feb. 21. 1989. Other suitable devices include dry powder inhalers such as Rotahaler® (Glaxo), Discus® (Glaxo), Spiros^{™} inhaler (Dura Pharmaceuticals), and the Spinhaler® (Fisons). Also useful is the multiple dose DPI system (MDDPI) which allows delivery of more than one therapeutic dose. Such devices are available from AstraZeneca, GlaxoWellcome, and SkyePharma. Also suitable are devices which employ the use of a piston to provide air for either entraining powdered medicament, lifting medicament from a carrier screen by passing air through the screen, or mixing air with powder medicament in a mixing chamber with subsequent introduction of the powder to the patient through the mouthpiece of the device, such as described in Mulhauser. P., *et al,* U.S. Patent No. 5,388,572, Sept. 30, 1997.

An inhaleable PEG-retinoid composition may also be delivered using a pressurized, metered dose inhaler (MDI), e.g., the Ventolin® metered dose inhaler, containing a solution or suspension of drug in a pharmaceutically inert liquid propellant, e.g., a chlorofluorocarbon or fluorocarbon, as described in Laube, *et al.,* U.S. Patent No. 5,320,094, June 14,1994, and in Rubsamen, R.M., *et al.* U.S. Patent No. 5,672,581 (1994). MDI devices are available from suppliers such as 3M Corporation, Aventis, Schering Plough and Vectura.

Alternatively, the PEG-retinoids described herein may be dissolved or suspended in a solvent, e.g., water or saline, and administered by nebulization. Nebulizers for delivering an aerosolized solution include the AERx^{™} (Aradigm), the Ultravent® (Mallinkrodt), the Pari LC Plus^{™} or the Pari LC Star^{™} (Pari GmbH, Germany), the DeVilbiss Pulmo-Aide, and the Acorn II® (Marquest Medical Products).

Another type of device that may be used to deliver a conjugate of the invention to the lung is a liquid spray device supplied, e.g., by Aradigm Corporation.

Alternatively, an electrohydrodynamic (EHD) aerosol device may be used to deliver a retinoid conjugate to the lung.

As previously described, the polymer-retinoid conjugates described herein can also be administered parenterally by intravenous injection, or less preferably by intramuscular or by subcutaneous injection. Precise components of such formulations can be readily determined by one skilled in the art. Suitable formulation types for parenteral administration include ready-for-injection solutions, dry powders for combination with a solvent prior to use, suspensions ready for injection, dry insoluble compositions for combination with a vehicle prior to use, emulsions and liquid concentrates for dilution prior to administration. For instance, an injectable solution of a PEG-retinoid composition of the invention may include the composition dissolved in an aqueous vehicle such as aqueous sodium chloride, Ringers solution, a dextrose-injection solution, lactated Ringers solution and the like, and may include one or more pharmaceutically acceptable compatible excipients or additives as described above.

### VII. Utility

The polymer conjugates of the invention can be used to treat any condition responsive to retinoic acid in mammals, including humans. Conditions responsive to retinoid therapy and that may be treated or ameliorated by administration of a conjugate in accordance with the invention include skin conditions such as acne, prostate cancer, leukemia, breast cancer, chronic bronchitis, asthma, emphysema, and aerodigestive cancers such as cancers of the head and neck. A preferred condition for treatment is emphysema, particularly by pulmonary administration of an inhaleable formulation of a polymer conjugate of the invention.

Pulmonary administration approaches can provide for the direct localized delivery of a retinoid such as ATRA to the target tissue. Moreover, the conjugates of the invention, when delivered directly to the lung, have been shown to reside in measurable concentrations for a prolonged period in lung tissue rather than being rapidly absorbed into the circulation, further demonstrating the usefulness of this approach.

Generally, a method of treatment or prophylaxis comprises administering to a mammal in need thereof a therapeutically effective amount of a polymer conjugate of retinoic acid as described above. The therapeutically effective dosage amount of any specific conjugate will vary somewhat from conjugate to conjugate, patient to patient, and will depend upon factors such as the condition and size of the patient, the loading capacity of the polymer conjugate, and the route of delivery. Usual patient dosages of cis retinoids when administered either orally or by injection range from about 0.1 microgram and about 10 milligrams, preferably between about 1.0 microgram and about 1.0 milligram, and more preferably between about 100 micrograms and about 300 micrograms. Administration of a cis-retinoid conjugate into the lung may reduce the required dose between about 2-fold to about 100 fold; thus appropriate dosage ranges for the conjugates of the invention can be calculated accordingly; based upon the dose of retinoid rather than the overall dose of conjugate delivered.

The following examples illustrate, but in no way are intended to limit the scope of the present invention.

### EXAMPLES

### Materials and Methods

Retinoic acid was obtained from Aldrich (St. Louis, MO).

All PEG reagents referred to in the appended examples are available from Shearwater Corporation of Huntsville, AL.

All ¹HNMR data was generated by a 300 or 400 MHz NMR spectrometer manufactured by Bruker.

Particle size measurements (Horiba): Mass median diameters (MMD) of the powders were measured using a Horiba CAPA-700 particle size analyzer (Horiba Instruments inc., Irvine, CA). Measurements were based upon centrifugal sedimentation of dispersed particles in suspending medium. Mass median diameter, which is based on the particle's Stokes' diameter, was calculated using the particle density and the density and viscosity of the suspending medium.

The density of the powder was set as 1.5 g/cm³ for all powders. (This nominal value was used for all powders analyzed and is within a range that is typical for spray dried powders). Particle size measurements were conducted with about 5 - 10 mg powder suspended in 5 ml Sedisperse A-11 (Micromeritics, Norcross, GA) and dispersed by sonication for 10 minutes. The range over which particle size data was gathered was set to 0.4 to 10.0 µm.

### Aerodynamic Particle Size Measurements

Andersen Cascade Impactor: An Andersen cascade impactor (a sieve-like apparatus with a series of stages that capture particles on plates by inertial impaction according to their size) was used to determine the MMAD and particle size distribution of aerosolized powder formulations in an air stream. The plates were weighed before and after testing and the mass of powder deposited on the plate of each stage was determined. Unless otherwise indicated, studies were undertaken using a traditional Andersen cascade impactor having eight stages (from top to bottom stages 0 to 7) with cut-off sizes ranging from 9.0 to 0.4 µm, and a final filter stage that traps particles < 0.4 µm when operated at a flow rate of 28.3 L/min. The device test set-up was similar to the ED test, except that the cascade impactor and a USP (United States Pharmacopia) throat (USP 23, chapter <601>) were attached to the device mouthpiece rather than to a filter. Multiple dispersions were typically conducted for each cascade impaction run to achieve gravimetrically accurate data.

The following examples demonstrate the preparation of polymer conjugates of the exemplary retinoids, cis and trans-retinoic acid. Conjugates were prepared using PEGs of various geometries and molecular weights. Exemplary conjugates having linear, forked and branched geometries were prepared with overall PEG molecular weights ranging from 2kD to 20 kD. The representative conjugates were synthesized to contain an ester linkage coupling the polymer arms to the retinoid moiety. Hydrolysis data demonstrating the hydrolytically degradable linkage feature of the conjugates in both buffer and serum are provided in Examples 9 and 10.

### Example 1

### Preparation of mPEG (5 kDa) ATRA Ester Conjugate

Synthesis of an exemplary PEG-retinoic acid conjugate utilizing a linear PEG having a molecular weight of 5 kD is described below.

In a round-bottom flask, 400 mg of ATRA (1.33 mmole, Aldrich) was dissolved in 60 ml of anhydrous benzene. To this solution was added 3 ml of oxalyl chloride in methylene chloride (2 M). The solution was stirred for two hours under argon in the absence of light. The solvent was then removed by rotary evaporation and the residue was further dried under vacuum. To the dry residue was added 3.2 grams of dry methoxy-PEG (5 kDa) in 60 ml of anhydrous benzene followed by 200 mg of dimethylaminopyridine (DMAP). The solution was stirred at room temperature overnight. The precipitate was removed by filtration, the filtrate was evaporated under vacuum and the residual syrup was added to 80 ml of ethyl ether. The resulting precipitated product was collected by filtration, washed with ether, and dried under vacuum. The product was further purified by preparative HPLC on a C4 column (Delta-Pak 15Um 100 A, 25x100mm) eluted with water/acetonitrile gradient. ¹H NMR(DMSO-d₆): δ 3.5 (br m, PEG), 4.15 (t, PEGOCH₂CH₂OCO-ATRA), 5.8-7.1 (M, H in double bond) 1.01 (s, 2CH₃), 1.68 (s, CH₃), 1.99 (s, CH₃), 2.30 (s, CH₃).

### Example 2

### Preparation of mPEG (20 kDa) ATRA Ester Conjugate

Synthesis of an exemplary conjugate utilizing a linear PEG having a molecular weight of 20 kD is described below.

In a round bottom flask, 300 mg of ATRA (1 mmole, Aldrich) was dissolved in 20 ml of anhydrous benzene. To this solution was added 2.2 ml of oxalyl chloride in methylene chloride (2 M). The solution was stirred for two hours in the absence of light. The solvent was then removed by rotary evaporation and the residue was further dried under vacuum. To the dry residue was added 10 grams of dry mPEG 20 kDa in 120 ml of mixture of anhydrous methylene chloride and benzene (1/1) followed by 150 mg of dimethylaminopyridine (DMAP). The solution was stirred at room temperature overnight. The precipitate was removed by filtration, the filtrate evaporated under vacuum, and the residual syrup was added to 300 ml of ethyl ether. The precipitated product was collected by filtration, washed with ether, and dried under vacuum. The product was further purified by preparative HPLC on a C4 column (Delta-Pak 15Um 100 A, 25x100mm) eluted with water/acetonitrile gradient. ¹H NMR(DMSO-d₆): δ 3.5 (br m, PEG), 4.15 (t, PEGOCH₂CH₂OCO-ATRA), 5.8-7.1 (M, H in double bond) 1.01 (s, 2CH₃), 1.68 (s, CH₃), 1.99 (s, CH₃), 2.30 (s, CH₃).

### Example 3

### Preparation of mPEG (2 kDa) ATRA Ester Conjugate

Synthesis of an exemplary conjugate utilizing a linear PEG having a molecular weight of 2 kD is described below.

In a round bottom flask, 200 mg of ATRA(0.66 mmole, Aldrich) was dissolved in 20 ml of anhydrous benzene. To this solution was added 1.5 ml of oxalyl chloride in methylene chloride (2 M). The solution was stirred for two hours under argon in the absence of light. The solvent was then removed by rotary evaporation and the residue was further dried under vacuum. To the dry residue was added 0.64 g of dry mPEG (monomethyl ether of PEG, methoxy-PEG-OH) 2kDa in 20 ml of anhydrous benzene followed by 120 mg of dimethylaminopyridine (DMAP). The solution was stirred at room temperature overnight. The precipitated product was removed by filtration, the filtrate evaporated under vacuum, and the residual syrup added to 60 ml of ethyl ether. The precipitate was collected by filtration, washed with ether, and dried under vacuum. ¹H NMR(DMSO-d₆): δ 3.5 (br m, PEG), 4.15 (t, PEGOCH₂CH₂OCO-ATRA), 5.8-7.1 (M, H in double bond) 1.01 (s, 2CH₃), 1.68 (s, CH₃), 1.99 (s, CH₃), 2.30 (s, CH₃).

### Example 4

### Preparation of PEG (20kDa) di-ATRA Ester Conjugate

ATRA-C(O)-O-PEG 20 kD-O-(O)C-ATRA

Synthesis of an exemplary conjugate utilizing a homobifunctional linear PEG reactant (PEG diol) having a molecular weight of 20 kD is described below. The resulting conjugate is characterized as having a dumbbell structure.

In a round bottom flask, 300 mg of ATRA(1 mmole, Aldrich) was dissolved in 20 ml of anhydrous benzene. To this solution was added 2.2 ml of oxalyl chloride in methylene chloride (2 M). The solution was stirred for two hours under argon in the absence of light. The solvent was then removed by rotary evaporation and the residue was further dried under vacuum. To the dry residue was added 5 g of dry PEG diol, 20 kDa, in 20 ml of anhydrous benzene followed by 150 mg of dimethylaminopyridine (DMAP). The solution was stirred at room temperature overnight. The precipitated product was removed by filtration, the filtrate evaporated under vacuum, and the residual syrup was added to 120 ml of ethyl ether. The precipitate was collected by filtration, washed with ether, and dried under vacuum. ¹H NMR(DMSO-d₆): δ 3.5 (br m, PEG), 4.15 (t, PEGOCH₂CH₂OCO-ATRA), 5.8-7.1 (M, H in double bond) 1.01 (s, 2CH₃), 1.68 (s, CH₃), 1.99 (s, CH₃), 2.30 (s, CH₃).

### Example 5

### Preparation of a 4-arm-PEG 20 kDa ATRA Ester Conjugate

C-[CH₂O-PEG-O(O)C-ATRA]₄

Synthesis of an exemplary branched or multi-armed conjugate characterized by four PEG arms each having an approximate molecular weight of about 5 kD and extending from a central pentaerythritol core is provided below. Thus, the overall structure of the conjugate is characterized by a central core from which extend four PEG arms each with a retinoic acid coupled to the terminus by an ester linkage, and having an overall molecular weight of about 20 kD.

In a round bottom flask, 200 mg of ATRA(0.66 mmole, Aldrich) was dissolved in 20 ml of anhydrous benzene. To this solution was added 2.2 ml of oxalyl chloride in methylene chloride (2 M). The solution was stirred for two hours under argon in the absence of light. The solvent was then removed by rotary evaporation and the residue was further dried under vacuum. To the dry residue was added 1.6 g of dry 4-arm PEG 20 kDa, C-[CH₂O-PEG-OH]₄ (Shearwater Corporation, Catalogue 2001, Polyethylene Glycol and Derivatives for Biomedical Applications, page 5) in 20 ml of anhydrous benzene and then 120 mg of dimethylaminopyridine (DMAP). The solution was stirred at room temperature overnight. The precipitated product was removed by filtration, the filtrate evaporated under vacuum, and the residual syrup added to 60 ml of ethyl ether. The precipitate was collected by filtration, washed with ether, and dried under vacuum. ¹H NMR(DMSO-d₆): δ 3.5 (br m, PEG), 4.15 (t, PEGOCH₂CH₂OCO-ATRA), 5.8-7.1 (M, H in double bond) 1.01 (s, 2CH₃), 1.68 (s, CH₃), 1.99 (s, CH₃), 2.30 (s, CH₃).

### Example 6

### Preparation of Benzyloxy-PEG 5 kDa ATRA Ester Conjugate

Synthesis of an exemplary conjugate utilizing a linear PEG having a molecular weight of 5 kD and end capped with a benzyloxy group is described below.

In a round bottom flask, 50 mg of ATRA(0.166 mmole, Aldrich), 0.7 g of benzyloxy-PEG-OH 5 kDa, 40 mg of DCC, 30 mg of HOBT and 10 mg DMAP were dissolved in 10 ml of anhydrous methylene chloride. The solution was stirred overnight at room temperature under argon in the absence of light. The solvent was then removed by rotary evaporation and the residue was partially dissolved in 10 ml of 1,4-dioxane. The insoluble material was removed by filtration and the solvent partially removed under vacuum. The resulting syrup was added to 60 ml of ethyl ether. The resulting precipitated product was collected by filtration, washed with ether, and dried under vacuum. ¹H NMR(DMSO-d₆): δ 3.5 (br m, PEG), 4.15 (t, PEGOCH₂CH₂OCO-ATRA), 4.48 (s, C₆H₅-CH₂-), 7.32 (m, C₆H₅-CH₂-), 5.8-7.1 (M, H in double bond) 1.01 (s, 2CH₃), 1.68 (s, CH₃), 1.99 (s, CH₃), 2.30 (s, CH₃).

### Example 7

### Preparation of 4-arm-PEG 10 kDa ATRA Ester Conjugate

C-[CH₂O-PEG-O(O)C-ATRA]₄

Synthesis of an exemplary branched or multi-armed conjugate characterized by four PEG arms each having an approximate molecular weight of about 2.5 kD and extending from a central pentaerythritol core is described below. The overall structure of the conjugate is characterized by a central core from which extend four PEG arms each with a retinoic acid coupled to the terminus by an ester linkage, and having an overall molecular weight of about 10 kD.

In a round bottom flask, 200 mg of ATRA (0.66 mmole, Aldrich) was dissolved in 20 ml of anhydrous benzene. To this solution was added 100 µl of oxalyl chloride. The solution was stirred for two hours under argon in the absence of light. The solvent was then removed by rotary evaporation and the residue was further dried under vacuum. To the dry residue was added 1 g of dry 4-arm PEG 10 kDa, C-[CH₂O-PEG-OH]₄. in 20 ml of anhydrous benzene and 100 mg of dimethylaminopyridine (DMAP). The solution was stirred at room temperature overnight. The precipitated product was removed by filtration, the solvent removed under vacuum, and the syrup added to 60 ml of ethyl ether. The precipitate was collected by filtration, washed with ether, and dried under vacuum. ¹H NMR(DMSO-d₆): δ 3.5 (br m, PEG), 4.15 (t, PEGOCH₂CH₂OCO-ATRA), 5.8-7.1 (M, H in double bond) 1.01 (s, 2CH₃), 1.68 (s, CH₃), 1.99 (s, CH₃), 2.30 (s, CH₃).

### Example 8

### Preparation of Forked PEG 5kDa-ATRA Ester Conjugate

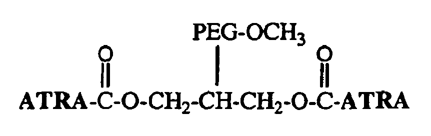

In this example, synthesis of a forked polymer conjugate having a central PEG chain from which extend two ATRA moieties extending from a hydrolytically stable branch point in the polymer or polymer linker (i.e., the CH group) is described. ATRA is coupled to the forked polymer structure via hydrolyzable ester linkages.

In a round bottom flask, 300 mg of all-trans retinoic acid (1 mmole, Aldrich) was dissolved in 20 ml of anhydrous benzene. To the resulting solution was added 2.2 ml of oxalyl chloride in methylene chloride (2 M) at 0°C. The solution was stirred for two hours under argon in the absence of light. The solvent was then removed by rotary evaporation and the residue was further dried under vacuum. To the dry residue was added 1.5 g of dry 2-mPEGyloxy-1,3-propanediol (5 kDa) in 20 ml of anhydrous benzene followed by 150 mg of dimethylaminopyridine (DMAP). The solution was stirred at room temperature overnight. The precipitated product was removed by filtration, the filtrate evaporated under vacuum, and the residue syrup was added to 120 ml of ethyl ether. The precipitate was collected by filtration, washed with excess ether, and dried under vacuum. ¹H NMR(DMSO-d₆): δ 3.5 (br m, PEG), 4.15 (dxd, MeO-PEGOCH(CH₂OCO-ATRA)₂, 5.8-7.1 (M, H in double bond) 1.01 (s, 2CH₃), 1.68 (s, CH₃), 1.99 (s, CH₃), 2.30 (s, CH₃).

### Example 9

### Hydrolysis rate of the ester of mPEG 5 kDa ATRA ester conjugate in buffer

The rate of hydrolysis in buffer of an exemplary PEG-ATRA conjugate in accordance with the invention was determined at two different temperatures, room temperature and body temperature.

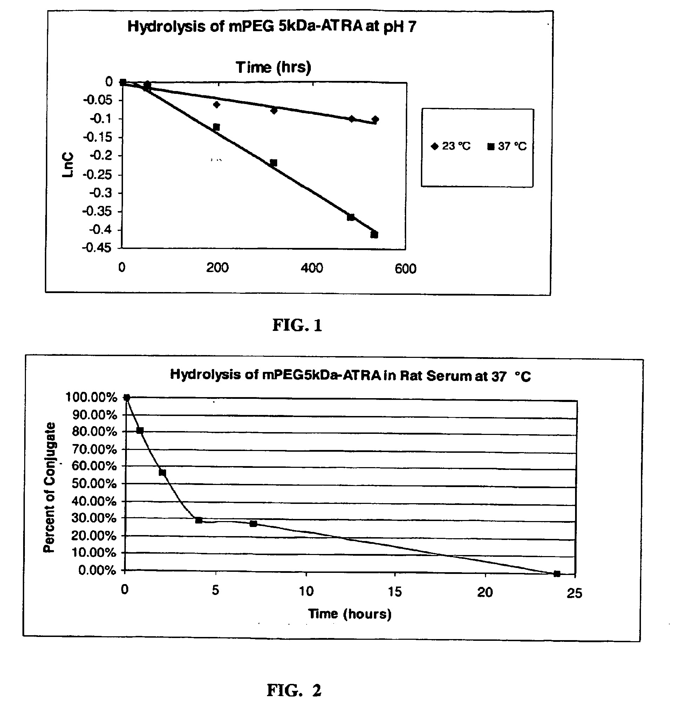

MPEG 5 kDa ATRA (see Example 1) was dissolved in phosphate buffer (0.1M, pH 7.0) at a concentration of 0.5 wt% at temperatures of 23 °C and 37 °C. At timed intervals, aliquots were removed for analysis by analytical reverse phase HPLC. The hydrolysis half-lives were obtained using pseudo-first-order kinetics. As shown in **Fig. 1,** the half-life (t ½) of the hydrolysis at 23°C was about 3500 hours, while the half-life at 37 °C was about 877 hours. These results demonstrate the hydrolyzable nature of these particular ester-coupled conjugates to release the parent retinoid compound. Thus, these conjugates can be characterized as water-soluble prodrugs forms of the retinoid.

### Example 10

### Hydrolysis study mPEG 5kDa-ATRA ester conjugate in rat serum

To supplement the buffer data from Example 9 above, the rate of hydrolysis of the same exemplary PEG-ATRA conjugate in rat serum was determined at 37 °C to provide an estimation of the hydrolysis rate under conditions more closely modeling those encountered by such conjugates in the body.

mPEG 5kDa-ATRA (80 mg) (see Example 1) was dissolved in 5 ml of rat serum and the resulting solution was incubated at 37 °C. At timed intervals. 0.7 ml of the ATRA/serum mixture was withdrawn and extracted twice with 2 ml of dichloromethane. The dichloromethane extract was dried with Na₂SO₄, filtered, and evaporated under vacuum. Water was added to the dried residue and the resulting mixture was filtered. The filtrate was analyzed for PEG ATRA and PEG by reverse phase HPLC (Betasil C18, 100x2,). As shown in **Fig. 2**, the half-life (t½) of the hydrolysis was approximately 2.5 hours.

Again, these results further support the hydrolyzable nature of ester conjugates in accordance with the invention, and over a course of time that lends itself to pulmonary adminstration of such conjugates. Such conjugates will likely hydrolyze over an extended period of hours when present in the lung to release the parent retinoid. Such profiles are particularly attractive for inhalation therapy via localized treatment of diseases of the respiratory tract.

### Example 11

### Recovery of ATRA and mPEG (5 kD) ATRA Ester Conjugate in Plasma and Lung Tissue After Intratracheal Instillation in Male Rats

The following study in rats was conducted to determine plasma and lung tissue concentrations of non-PEGylated all-trans retinoic acid (ATRA control) versus a PEGylated all-trans retinoic acid, mPEG (5kD)ATRA Ester Conjugate, after intratracheal instillation to male rats.

Animals: Hilltop Lab Animals Inc (P.O. Box 183, Scottdale, PA 15683) supplied pre-cannulated (jugular vein catheter [JVC]) Sprague Dawley Rats (350-375 grams). The test system included 2 male rats randomly chosen for each of the 6 test groups.

ATRA and PEG-ATRA Samples: Non-PEGylated All-Trans Retinoic Acid was supplied as a powder containing 50% ATRA by weight (Control Article). The powder was stored at -20°C and protected from light. Methoxy PEG (5kD) ATRA ester conjugate (Test Article) was provided as a powder containing 5% ATRA by weight which was stored at -20°C and protected from light.

Stock Solutions: Stock Solutions for dosing were prepared as follows.

ATRA Stock Solution/Control (2.0 mg/ml Stock): 3.0 mL of PBS was added to 6.0 mg of the ATRA powder. This solution was dosed as a suspension to the rats after sonication and vortexing.

Methoxy PEG (5kD) ATRA ester conjugate (20.0 mg/mL Stock): 3.0 mL of PBS was added to 60.0 mg of the methoxy PEG (5kD) ATRA ester conjugate powder.

Intratracheal Instillation: Administration of the control and test articles was achieved by intratracheal instillation under yellow lighting. The rats were lightly anesthetized using inhaled 3.0 - 5.0% isoflurane mixed with oxygen in a plexiglass anesthesia chamber. The dose was administered by inserting a gavage needle fitted with a 1 mL syringe into the mouth of the rat down the trachea to just above the carina. The dose was administered into the lungs utilizing this method, and then the gavage needle was removed. The animal was placed back in its cage and allowed to recover from the anesthesia on its own after the dosing procedure.
The rats received the following doses:

| **Group No.** | **Control/Test Article** | **Number of Animals/Gender** | **Time of Sacrifice** | **Total Daily Dose of All-trans retinoic acid** |
|---|---|---|---|---|
| | | | **(min)** | **(µg/animal)** |
| 1 | **All-Trans Retinoic Acid** | **2M** | 5 | 300 |
| 2 | **All-Trans Retinoic Acid** | **2M** | 60 | 300 |
| 3 | **All-Trans Retinoic Acid** | **2M** | 120 | 300 |
| 4 | **5K PEG All-Trans Retinoic Acid** | **2M** | 5 | 300 |
| 5 | **5K PEG All-Trans Retinoic Acid** | **2M** | 60 | 300 |
| 6 | **5K PEG All-Trans Retinoic Acid** | **2M** | 120 | 300 |

Rats were dosed IT with 354µg (1.2µmole) of RA or 300µg (1µmole) ATRA equivalent of mPEGSK-ATRA.

Blood Collection: Predose blood samples of approximately 3.0 ml were collected from the jugular vein catheter into heparinized plasma tubes. At the designated sacrifice time for each animal a surgical plane of anesthesia was induced by using inhaled 3.0 - 5.0% isofluorane mixed with oxygen. Once the surgical plane anesthesia was reached, a terminal blood draw was collected. The plasma samples were protected from sunlight and white light and stored frozen at -20°C.

Lung Harvest: The diaphragm was punctured following the terminal blood draw. The trachea of the rat was exposed and the ribs were separated allowing access to the lungs. The heart and lungs were removed enbloc. Once the lungs were excised the heart and any connective tissue still remaining was removed. The lungs were protected from sunlight and white light, wrapped in aluminum foil and snap frozen using liquid nitrogen, and stored at -80°C.

ATRA and PEG-ATRA were extracted from whole rat lung by first weighing and transferring the rat lung to an amber vial and cutting the lung into small pieces. HPLC grade water was then added to the cut-up lung tissue, the sample was placed into an ice bath, and the tissue was then homogenized and sonicated. The resulting sample was transferred to a centrifuge tube to which was added ethanol, and the sample vortexed for approximately one minute, followed by centrifugation for 30 minutes at 14,000 RPM at 2-8°C_ The sample was then analyzed by HPLC.

Sample Analysis: Plasma and lung tissue samples were analyzed for all-trans retinoic acid concentrations by reverse-phase high performance liquid chromatography (HPLC) procedures for the lung tissue and by a liquid chromatography tandem mass spectrophotometer (LC-MS-MS) technique for the plasma samples. HPLC Analysis was conducted using a Waters 2690 HPLC instrument and a Vydac C18 4.6 x 250 mm column.

Standard solutions of various known concentrations of PEG-ATRA dissolved in water and ATRA dissolved in ethanol were prepared and utilized to form lung matrix samples prepared from homogenized rat lung preparations as described above. To prepare lung matrix samples for analysis, to homogenized samples of whole rat lung as described above were added known volumes of the above standard solutions. Samples were assayed to provide an indication of the content of retinoid in lung tissue at various time points after IT administration of both ATRA and PEG-ATRA.

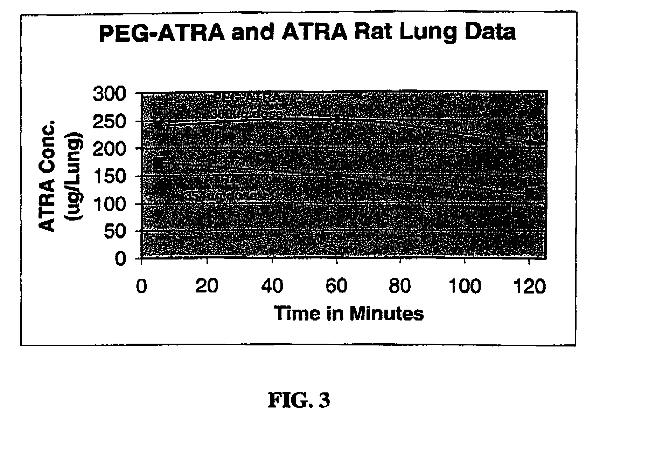

The results (summarized graphically in FIG. 3) indicated that sustained and reasonably high levels of ATRA corresponding to PEG ATRA were achieved locally in the lung tissue subsequent to delivery to the lung, and that high systemic levels of retinoid were minimized. More specifically, the HPLC data suggests that PEG-ATRA is retained in the lung at greater than 70% of the dose for at least up to 2 hours post administration. ATRA (not PEGylated) was also held up in the lung **(Fig. 3),** but to a lesser degree than its pegylated counterpart.

Analysis of the rat plasma at the one-hour timepoint showed that the plasma retinoic acid (ATRA) concentration was approximately 145 ng/ml for the non-PEGylated-ATRA formulation whereas the plasma retinoic acid concentration was approximately 65 ng/ ml for the PEGylated-ATRA formulation. These results along with the lung tissue concentrations shown in FIG. 3 indicate that polymer modified retinoids such as PEGylated ATRA are retained in the lung over a longer period of time and at significantly higher concentrations than their non polymer-modified counterparts.

### Example 12

### Preparation of mPEG (5 kDa) Conjugate of 13-cis-Retinoic Acid (13-cis-RA) through an Ester Linkage

In a round-bottom flask, 156 mg of 13-cis-Retinoic Acid (1.33 mmole, Aldrich) was dissolved in 25 ml of anhydrous benzene. To this solution was added 1.9 ml of oxalyl chloride in methylene chloride (2 M). The solution was stirred for two hours under argon in the absence of light. The solvent was then removed by rotary evaporation at 40 °C and the residue was further dried under vacuum. To the dry residue was added 2 grams of dry methoxy PEG-OH (5 kDa) in 25 ml of anhydrous benzene followed by 125 mg of dimethylaminopyridine (DMAP). The solution was stirred at room temperature overnight. The precipitate was removed by filtration, the filtrate was evaporated under vacuum and the residual syrup was added to 50 ml of ethyl ether. The resulting precipitate was collected by filtration, washed with ether, and dried under vacuum. Yield: 1.99 g. ¹H NMR(DMSO-d₆): δ 3.5 (br m, PEG), 4.15 (t, PEGOCH₂CH₂OCO-13-cis-RA), 5.8-7.1 (M, H in double bond), 1.02 (s, 2CH₃), 1.69 (s, CH₃), 1.99 (s, CH₃).

### Example 13

### Spray Drying of ATRA

The following experiment was conducted to explore the features of a spray dried retinoic acid formulation.

All trans retinoic acid (2.997 g) was dissolved in 255 mL of ethanol to form a fine yellow-colored suspension (not all of the ATRA dissolved) which was then spray dried using a Bucci spray dryer operating at an inlet temperature of 98 °C and an outlet temperature of 71 °C using nitrogen as the feed gas. Spray dried powder was recovered in about 65% yield. However, a significant quantity of the brightly colored retinoic acid accumulated throughout the spray dryer apparatus during spray drying of the feed solution, presumably due to sublimation of the compound during the spray drying process.

The low melting and sublimation temperatures of the retinoids, and in particular ATRA, make spray drying of the unmodified compound somewhat problematic when using conventional spray drying conditions.

### Example 14

### Spray Dried PEG-Retinoic Acid Powder Suitable for Inhalation

An illustrative PEG-retinoid conjugate, mPEG (5kD) ATRA ester conjugate, was formulated as a dry powder for inhalation. For certain dry powder formulations, trileucine was used as an exemplary excipient to enhance aerosol properties and stability.

The inhaleable dry powder formulation was prepared as follows: 100 mg of purified mPEG5K-ATRA ester conjugate and 400 mg of trileucine (Bachem California Inc, USA Torrance, CA) were dissolved in 200 mL of HPLC grade water. The resulting solution contained 80% solids weight percent trileucine, 20% solids weight percent mPEG5K-ATRA with a total solids concentration of 0.25%.

The solution was then spray dried using a Buchi 190 spray dryer at a feed rate of approximately 3 to 5 mL/min, an inlet temperature of 69°C, an outlet temperature of 40°C, and a vacuum of -100 mbar. Due to the low melting temperature of the PEG-ATRA conjugate, an unusually low outlet temperature was employed.

The spray drying yield was 29%, and the spray dried powder was yellow in color and free flowing. Scanning electron micrographs (SEMs) of the particles showed a wrinkled morphology of particles sized at about 2 µm. The melting point of the powder, measured by differential scanning calorimetry, was 200°C. The melting point of the mPEG5K-ATRA ester conjugate is around 53 °C.

Aerosol data for the powder was determined.

| | |
|---|---|
| ED | 74%, |
| MMAD | 3.1µm, |
| FPM < 5.0µm | 54% |
| FPM < 3.3µm | 39% |

| | |
|---|---|
| (FPM stands for fine particle mass which is the weight of the dry powder (or liquid droplets depending upon the formulation) smaller than the defined size, e.g., 3.3 microns) | |

A spray dried formulation composed of 20% by weight trileucine and 80% by weight mPEG5K-ATRA ester conjugate was similarly prepared. The spray dried powder had the following properties: ED 27%, MMAD 7.0 µm, FPM < 3.3µm 3%. The melting point of the powder, measured by differential scanning calorimetry, was around 53 °C.

### Example 15

### Spray Dried PEG-Retinoic Acid Powder Suitable for Inhalation

An illustrative PEG-retinoid conjugate, mPEG (5kD) ATRA ester conjugate, was formulated in an alternative fashion as a dry powder for inhalation.

A PEG-ATRA formulation composed of 60% PEG-RA / 37% DSPC / 3% CaCl₂ was prepared as follows. 183 mg of DSPC and 17 mg of calcium chloride were added to 22.9g of hot DI water (>70°C). DSPC and CaCl₂ were dispersed into the hot DI water using a homogenizer operated at 10,000 rpm for 5 minutes. 10.9 g of PFOB (perfluorooctylbromide) was then added to the mixture and the mixture was further mixed at a mixing rate of 12,000 rpm for 5 minutes. The emulsion was further processed through a homogenizer at 18,000 psi for several additional passes. 300 mg of mPEG5K-ATRA (Example 1) was added to the emulsion. The emulsion was spray dried using the Buchi B-191 spray dryer at a feed rate of 2.0 mL/min, an inlet temperature of 85°C, an outlet temperature of 66°C, and a vacuum of -29 mbar.

The spray drying yield was 17%. The resulting spray dried powder composition was yellow in color and was free flowing. Scanning electronn micrographs SEM revealed particles that were somewhat non-spherical and less visibly porous than expected.

Aerosol data for the resulting powder was generated.

| | |
|---|---|
| Impactor ED | 63%, |
| MMAD | 3.5µm, |
| FPM < 5.0µm | 86% |
| FPM < 3.3µm | 44% |

Both of the above examples, Examples 15 and 16, demonstrate the preparation of dry powder formulations of pegylated retinoids having aerosol properties (e.g., dispersibilities and aerodynamic diameters) that make them particularly well suited or advantageous for pulmonary delivery to the lung.

Many modifications and other embodiments of the invention will come to mind to one skilled in the art to which this invention pertains having the benefit of the teachings presented in the foregoing descriptions and the associated drawings. Therefore, it is to be understood that the invention is not to be limited to the specific embodiments disclosed.

Although specific terms are employed herein, they are used in a generic and descriptive sense only and not for purposes of limitation.

## Claims

1. A polymer-retinoid conjugate composition suitable for pulmonary administration, said composition comprising a retinoid covalently bonded to a water-soluble and non-peptidic polymer selected from the group consisting of poly(alkylene glycol), poly(oxyethylated polyol), poly(olefinic alcohol), poly(vinylpyrrolidone), poly(hydroxyalkylmethacrylamide), poly(hydroxyalkylmethacrylate), poly(α-hydroxy acid), poly(vinyl alcohol), polyphosphazene, polyoxazoline, poly(N-acryloylmorpholine), and copolymers, terpolymers, and mixtures thereof.

2. The composition of claim 1, wherein said polymer is polyethylene glycol.

3. The composition of claim 1, wherein said retinoid is selected from the group consisting of 13-cis retinoic acid, all trans retinoic acid, 9-cis retinoic acid, 11-cis retinoic acid, and retinol.

4. The composition of claim 3, wherein said retinoid is all-trans retinoic acid.

5. The retinoid composition of claim 3, wherein said retinoid is a cis-retinoic acid.

6. The composition of claim 1, wherein said polymer retinoid conjugate is water-soluble.

7. The composition of claim 1 in liquid or dry form.

8. An aerosol comprising the composition of claim 1.

9. The composition of claim 1 in an inhaler device.

10. The composition of claim 1, which when aerosolized, is **characterized by** a mass median aerodynamic diameter (MMAD) of less than 10 µm (microns).

11. The composition of claim 1, which when aerosolized, is **characterized by** a MMAD of less than 5 µm (microns).

12. The composition of claim 1 further comprising a pharmaceutically acceptable excipient.

13. The composition of claim 1 further comprising a free radical scavenger.

14. The composition of claim 7, wherein said composition is a dry powder.

15. The composition of claim 14 **characterized by** an emitted dose of at least 30 percent.

16. A spray-dried composition of claim 1.

17. A unit dosage form comprising the composition of claim 1 for use in an inhaler device.

18. The composition of claim 1 wherein said water soluble and non-peptidic polymer is covalently bonded to the retinoid via a hydrolytically unstable linkage.

19. The composition of claim 18 wherein said hydrolytically unstable linkage is selected from the group consisting of ester, thiolester (-C(O)-S) and amide.

20. The composition of claim 1 wherein said water soluble and non-peptidic polymer is covalently bonded to the retinoid via a hydrolytically stable linkage.

21. The composition of claim 1 wherein said water soluble and non-peptidic polymer has an average molecular weight from 500 daltons to 100,000 daltons.

22. The composition of claim 21 wherein said water soluble and non-peptidic polymer has an average molecular weight from 750 daltons to 40,000 daltons.

23. The composition of claim 2 wherein said polyethylene glycol is end-capped.

24. The composition of claim 2 wherein said polyethylene glycol is end-capped with an alkoxy group.

25. The composition of claim 2 wherein said polyethylene glycol is selected from the group consisting of linear polyethylene glycol, branched polyethylene glycol, forked polyethylene glycol and dumbbell polyethylene glycol.

26. The composition of claim 1 absent an agent necessary for solubilizing said retinoid in a carrier vehicle.

27. A polymer retinoid conjugate composition of any one of claims 1-26 for use as a medicament for pulmonary administration to a human subject.

28. The composition of claim 27 for treatment of chronic obstructive pulmonary disease.

29. Use of a polymer retinoid conjugate composition of any one of claims 1-26 for the preparation of a medicament for treating chronic obstructive pulmonary disease, wherein said medicament is to be administered by inhalation.

30. The polymer retinoid conjugate of claim 18, wherein said polymer is covalently attached, via a hydrolytically degradable linkage, to the carbonyl carbon of retinoic acid, and further wherein (i) if the polymer is a linear polyethylene glycol and the linkage is an ester linkage, then the molecular weight of the polyethylene glycol is at least 2,000 daltons, and (ii) if the polymer is a linear end-capped polyethylene glycol and the linkage is an amide linkage, then the polyethylene glycol has a molecular weight of at least 5,000 daltons.

31. The polymer retinoid conjugate of claim 30 wherein said hydrolytically degradable linkage is selected from the group consisting of carboxylate ester, amide, and thiolester.

32. The polymer retinoid conjugate of claim 30, wherein the polymer retinoid conjugate comprises the structure: 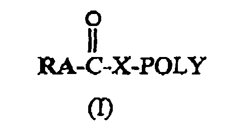 wherein RA together with the carbonyl group represents a retinoic acid moiety,
X is a heteroatom selected from O, N-H, and S, and
POLY is the water soluble, non-peptidic polymer,
wherein (i) if the POLY is a linear polyethylene glycol and X is an O, then the molecular weight of the POLY is at least 2,000 daltons, and (ii) if POLY is a linear end-capped polyethylene glycol and X is a N, then POLY has a molecular weight of at least 5,000 daltons.

33. The polymer retinoid conjugate of claim 32 selected from the group consisting of:

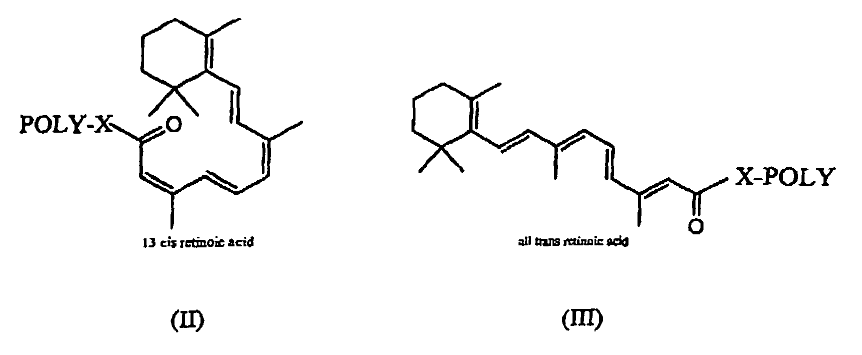

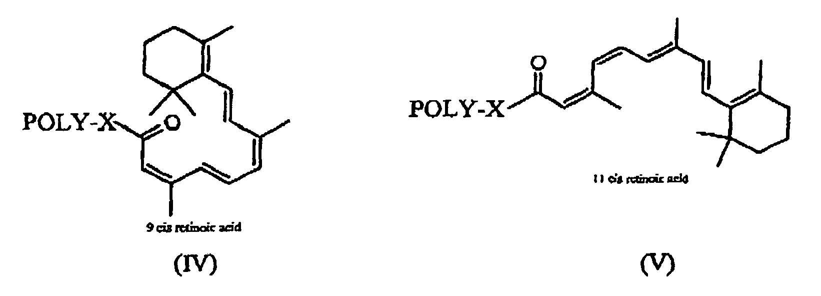

34. The polymer retinoid conjugate of Claim 33, wherein POLY is a poly(ethylene glycol).

35. The polymer retinoid conjugate of Claim 33, wherein X is O or S.

36. The polymer retinoid conjugate of Claim 33, wherein POLY has 2 to 300 termini.

37. The polymer retinoid conjugate of Claim 33, wherein said POLY is end-capped.

38. The polymer retinoid conjugate of claim 37, having the following structure: 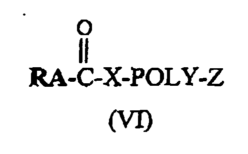 wherein Z is a functional group.

39. The polymer retinoid conjugate of Claim 38, wherein Z has the structure 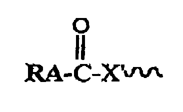 wherein X' together with the carbonyl is a hydrolytically degradable linkage and 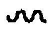 represents the point of attachment to POLY.

40. The polymer retinoid conjugate of Claim 38, wherein POLY is poly(ethylene glycol) and Z is methoxy.

41. The polymer retinoid conjugate of Claim 33 having the structure: 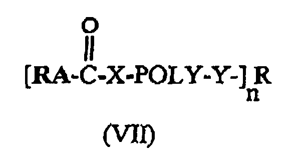 wherein:
n is an integer from 3 to 100;
R is a central core molecule;
X together with the adjacent carbonyl group is a hydrolytically degradable linkage;
Y is a linkage; and
each POLY is an independently selected water-soluble and non-peptidic polymer as defined in claim 1.

42. The polymer retinoid conjugate of Claim 41, wherein n ranges from 3 to 20.

43. The polymer retinoid conjugate of Claim 41, wherein Y is O, S or NH.

44. The polymer retinoid conjugate of Claim 41, wherein R is a residue of a central core molecule selected from the group consisting of glycerol, glycerol oligomers, pentaerythritol, sorbitol, and lysine.

45. The polymer retinoid conjugate of claim 32, wherein POLY is a polyethylene glycol selected from the group consisting of linear, branched, forked, or dumbbell PEG.

46. A method for preparing a retinoid formulation suitable for pulmonary administration, said method comprising the steps of:
covalently bonding a retinoid to a water soluble polymer as recited in Claim 1 to form a water soluble polymer retinoid conjugate,
preparing a pharmaceutical composition comprising said conjugate, wherein said composition is suitable for pulmonary administration, and
aerosolizing said composition.

47. The method of claim 46, wherein said preparing step comprises combining said conjugate with one or more excipients.

## Patentansprüche

1. Polymerretinoidkonjugatzusammensetzung, die für die Verabreichung an die Lungen geeignet ist, wobei die Zusammensetzung ein Retinoid umfasst, das kovalent an ein wasserlösliches und nichtpeptidisches Polymer gebunden ist ausgewählt aus der Gruppe bestehend aus Poly(alkylenglykol), poly(oxyethyliertem Polyol), poly(olefinischem Alkohol), Poly(vinylpyrrolidon), Poly(hydroxyalkylmethacrylamid), Poly(hydroxyalkylmethacrylat), Poly(α-hydroxysäure), Poly(vinylalkohol), Polyphosphazen, Polyoxazolin, Poly(N-acryloylmorpholin) und Copolymeren, Terpolymeren und Mischungen derselben.

2. Zusammensetzung nach Anspruch 1, wobei das Polymer Polyethylenglykol ist.

3. Zusammensetzung nach Anspruch 1, wobei das Retinoid aus der Gruppe ausgewählt ist bestehend aus 13-cis-Retinsäure, all-trans-Retinsäure, 9-cis-Retinsäure, 11-cis-Retinsäure und Retinol.

4. Zusammensetzung nach Anspruch 3, wobei das Retinoid all-trans-Retinsäure ist.

5. Zusammensetzung nach Anspruch 3, wobei das Retinoid eine cis-Retinsäure ist.

6. Zusammensetzung nach Anspruch 1, wobei das Polymerretinoidkonjugat wasserlöslich ist.

7. Zusammensetzung nach Anspruch 1 in flüssiger oder trockener Form.

8. Aerosol umfassend die Zusammensetzung nach Anspruch 1.

9. Zusammensetzung nach Anspruch 1 in einer Inhalatorvorrichtung.

10. Zusammensetzung nach Anspruch 1, die, wenn sie in ein Aerosol umgewandelt ist, durch einen mittleren aerodynamischen Massendurchmesser (MAMD) von weniger als 10 µm (Mikron) **gekennzeichnet** ist.

11. Zusammensetzung nach Anspruch 1, die, wenn sie in ein Aerosol umgewandelt ist, durch einen mittleren aerodynamischen Massendurchmesser (MAMD)von weniger als 5 µm (Mikron) **gekennzeichnet** ist.

12. Zusammensetzung nach Anspruch 1, des Weiteren eine pharmazeutisch akzeptable Trägersubstanz umfassend.

13. Zusammensetzung nach Anspruch 1, des Weiteren einen Radikalfänger umfassend.

14. Zusammensetzung nach Anspruch 7, wobei die Zusammensetzung ein trockenes Pulver ist.

15. Zusammensetzung nach Anspruch 14, **gekennzeichnet durch** eine emittierte Dosis von mindestens 30 Prozent.

16. Sprühgetrocknete Zusammensetzung nach Anspruch 1.

17. Einheitsdosis umfassend die Zusammensetzung nach Anspruch 1 zur Verwendung in einer Inhalatorvorrichtung.

18. Zusammensetzung nach Anspruch 1, wobei das wasserlösliche und nichtpeptidische Polymer durch eine hydrolytisch unbeständige Verknüpfung kovalent an das Retinoid gebunden ist.

19. Zusammensetzung nach Anspruch 18, wobei die hydrolytisch unbeständige Verknüpfung aus der Gruppe ausgewählt ist bestehend aus Ester, Thiolester(-C(O)-S) und Amid.

20. Zusammensetzung nach Anspruch 1, wobei das wasserlösliche und nichtpeptidische Polymer durch eine hydrolytisch beständige Verknüpfung kovalent an das Retinoid gebunden ist.

21. Zusammensetzung nach Anspruch 1, wobei das wasserlösliche und nichtpeptidische Polymer eine durchschnittliche Molmasse von 500 Dalton bis 100.000 Dalton aufweist.

22. Zusammensetzung nach Anspruch 21, wobei das wasserlösliche und nichtpeptidische Polymer eine durchschnittliche Molmasse von 750 Dalton bis 40.000 Dalton aufweist.

23. Zusammensetzung nach Anspruch 2, wobei das Polyethylenglykol endverkappt ist.

24. Zusammensetzung nach Anspruch 2, wobei das Polyethylenglykol mit einer Alkyoxygruppe endverkappt ist.

25. Zusammensetzung nach Anspruch 2, wobei das Polyethylenglykol aus der Gruppe ausgewählt ist bestehend aus linearem Polyethylenglykol, verzweigtem Polyethylenglykol, vergabeltem Polyethylenglykol und Hantel-Polyethylenglykol.

26. Zusammensetzung nach Anspruch 1 ohne Mittel, das zum Löslichmachen des Retinoids in einem Trägervehikel erforderlich ist.

27. Polymerretinoidkonjugatzusammensetzung nach einem der Ansprüche 1 - 26 zur Verwendung als Medikament für die Verabreichung an die Lungen bei einem menschlichen Patienten.

28. Zusammensetzung nach Anspruch 27 für die Behandlung einer chronischen Obstruktionslungenerkrankung.

29. Verwendung einer Polymerretinoidkonjugatzusammensetzung nach einem der Ansprüche 1 - 26 für die Herstellung eines Medikaments für die Behandlung einer chronischen Obstruktionslungenerkrankung, wobei das Medikament durch Inhalation verabreicht werden soll.

30. Polymerretinoidkonjugat nach Anspruch 18, wobei das Polymer durch eine hydrolytisch abbaubare Verknüpfung kovalent an den Carbonylkohlenstoff der Retinsäure angelagert ist und wobei des Weiteren die Molmasse des Polyethylenglykols, (i) wenn das Polymer ein lineares Polyethylenglykol und die Verknüpfung eine Esterverknüpfung ist, mindestens 2.000 Dalton beträgt und (ii) wenn das Polymer ein lineares, endverkapptes Polyethylenglykol und die Verknüpfung eine Amidverknüpfung ist, das Polyethylenglykol eine Molmasse von mindestens 5.000 Dalton aufweist.

31. Polymerretinoidkonjugat nach Anspruch 30, wobei die hydrolytisch abbaubare Verknüpfung aus der Gruppe ausgewählt ist bestehend Carboxylatester, Amid und Thiolester.

32. Polymerretinoidkonjugat nach Anspruch 30, wobei das Polymerretinoidkonjugat die Struktur: 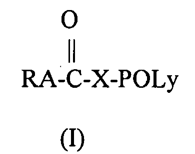 umfasst, wobei RA zusammen mit der Carbonylgruppe einen Retinsäureanteil darstellt,
X ein Heteratom ausgewählt unter O, N-H und S ist und
POLY das wasserlösliche, nicht peptidische Polymer ist,
wobei (i) wenn POLY ein lineares Polyethylenglykol und X ein O ist, die Molmasse des POLY mindestens 2.000 Dalton beträgt und (ii) wenn POLY ein lineares endverkapptes Polyethylenglykol und X ein N ist, POLY eine Molmasse von mindestens 5.000 Dalton aufweist.

33. Polymerretinoidkonjugat nach Anspruch 32 ausgewählt aus der Gruppe bestehend aus:

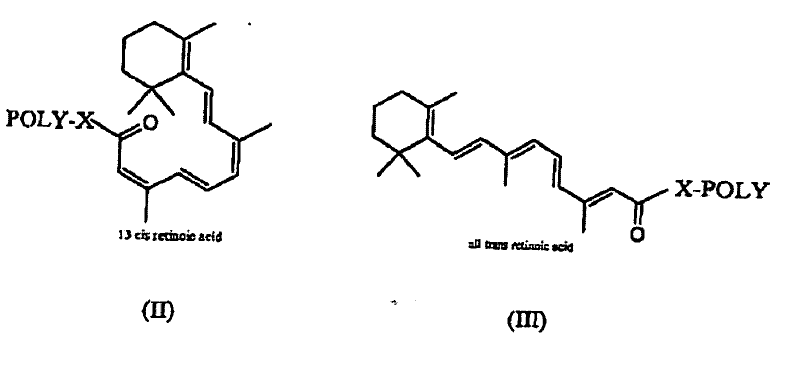

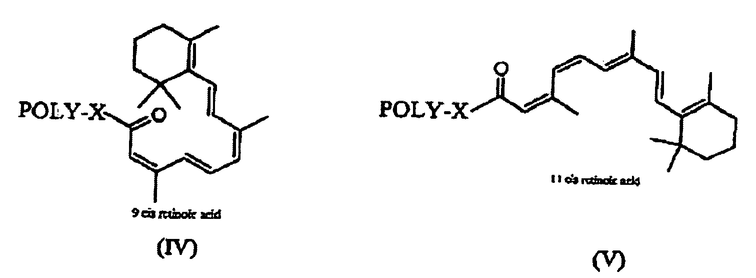

34. Polymerretinoidkonjugat nach Anspruch 33, wobei POLY ein Poly(ethylenglykol) ist.

35. Polymerretinoidkonjugat nach Anspruch 33, wobei X O oder S ist.

36. Polymerretinoidkonjugat nach Anspruch 33, wobei POLY 2 bis 300 Termini aufweist.

37. Polymerretinoidkonjugat nach Anspruch 33, wobei POLY endverkappt ist.

38. Polymerretinoidkonjugat nach Anspruch 37, das folgende Struktur aufweist: 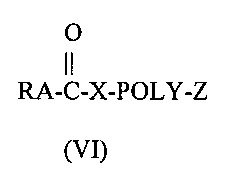 wobei Z eine funktionelle Gruppe ist.

39. Polymerretinoidkonjugat nach Anspruch 38, wobei Z die Struktur 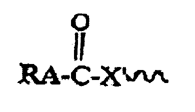 aufweist, wobei X' zusammen mit dem Carbonyl eine hydrolytisch abbaubare Verknüpfung ist und 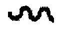 den Anlagerungspunkt an POLY darstellt.

40. Polymerretinoidkonjugat nach Anspruch 38, wobei POLY Poly(ethylenglykol) und Z Methoxy ist.

41. Polymerretinoidkonjugat nach Anspruch 33 mit der Struktur: 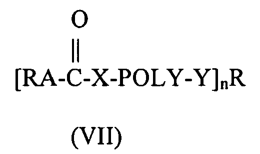 wobei:
n eine ganze Zahl von 3 bis 100 ist;
R ein mittiges Kernmolekül ist;
X zusammen mit der anliegenden Carbonylgruppe eine hydrolytisch abbaubare Verknüpfung ist;
Y eine Verknüpfung ist; und
jedes POLY ein unabhängig ausgewähltes wasserlösliches und nichtpeptidisches Polymer, wie in Anspruch 1 definiert, ist.

42. Polymerretinoidkonjugat nach Anspruch 41, wobei n im Bereich von 3 bis 20 liegt.

43. Polymerretinoidkonjugat nach Anspruch 41, wobei Y O, S oder NH ist.

44. Polymerretinoidkonjugat nach Anspruch 41, wobei R ein Rest eines mittigen Kernmoleküls ist ausgewählt aus der Gruppe bestehend aus Glycerin, Glycerinoligomeren, Pentaerythrit, Sorbit und Lysin.

45. Polymerretinoidkonjugat nach Anspruch 32, wobei POLY ein Polyethylenglykol ist ausgewählt aus der Gruppe bestehend aus linearem, verzweigtem, vergabeltem oder Hantel-PEG.

46. Verfahren für die Herstellung einer Retinoidrezeptur, die für die Verabreichung an die Lungen geeignet ist, wobei das Verfahren folgende Schritte umfasst:
das kovalente Binden eines Retinoids an ein wasserlösliches Polymer wie in Anspruch 1 angegeben unter Bildung eines wasserlöslichen Polymerretinoidkonjugats,
das Herstellen einer pharmazeutischen Zusammensetzung umfassend das Konjugat, wobei die Zusammensetzung für die Verabreichung an die Lungen geeignet ist, und
Umwandeln der Zusammensetzung in ein Aerosol.

47. Methode nach Anspruch 46, wobei der Herstellungsschritt das Kombinieren des Konjugats mit einer oder mehreren Trägersubstanzen umfasst.

## Revendications

1. Composition conjuguée polymère-rétinoïde convenant à l'administration par voie pulmonaire, ladite composition comprenant un rétinoïde lié covalentement à un polymère hydrosoluble et non peptidique sélectionné parmi le groupe comprenant le poly(alkylèneglycol), le poly(oxyéthylé-polyol), le poly(alcool oléfinique), la poly(vinylpyrrolidone), la poly(hydroxyalkylméthacrylamide), le poly(hydroxyalkylméthacrylate), le poly(acide α-hydroxy), le poly(alcool vinylique), le polyphosphazène, la polyoxazoline, la poly(N-acryloylmorpholine), et les copolymères, les terpolymères et des mélanges de ceux-ci.

2. Composition selon la revendication 1, dans laquelle ledit polymère est le polyéthylèneglycol.

3. Composition selon la revendication 1, dans laquelle ledit rétinoïde est sélectionné parmi le groupe comprenant l'acide 13-cis rétinoïque, l'acide all-trans rétinoïque, l'acide 9-cis rétinoïque, l'acide 11-cis rétinoïque et le rétinol.

4. Composition selon la revendication 3, dans laquelle ledit rétinoïde est l'acide all-trans rétinoïque.

5. Composition rétinoïde selon la revendication 3, dans laquelle ledit rétinoïde est un acide cis-rétinoïque.

6. Composition selon la revendication 1, dans laquelle ledit conjugué polymère-rétinoïde est hydrosoluble.

7. Composition selon la revendication 1 sous forme liquide ou desséchée.

8. Aérosol comprenant la composition selon la revendication 1.

9. Composition selon la revendication 1 dans un dispositif inhalateur.

10. Composition selon la revendication 1 qui, lorsque aérosolisée, se **caractérise en ce que** le diamètre aérodynamique médian massique (DAMM) est inférieur à 10 µm (microns).

11. Composition selon la revendication 1 qui, lorsque aérosolisée, se **caractérise en ce que** le DAMM est inférieur à 5 µm (microns).

12. Composition selon la revendication 1 comprenant, en outre, un excipient pharmaceutiquement acceptable.

13. Composition selon la revendication 1 comprenant, en outre, un antiradicalaire.

14. Composition selon la revendication 7, dans laquelle ladite composition est une poudre desséchée.

15. Composition selon la revendication 14, **caractérisée en ce qu'**une dose émise est d'au moins 30 pour cent.

16. Composition selon la revendication 1 desséchée par pulvérisation.

17. Forme pharmaceutique unitaire comprenant la composition selon la revendication 1, à utiliser dans un dispositif inhalateur.

18. Composition selon la revendication 1, dans laquelle ledit polymère hydrosoluble et non peptidique est lié covalentement au rétinoïde par une liaison hydrolitiquement instable.

19. Composition selon la revendication 18, dans laquelle ladite liaison hydrolitiquement instable est sélectionnée parmi le groupe comprenant l'ester, le thiolester (-C(O)-S) et l'amide.

20. Composition selon la revendication 1, dans laquelle ledit copolymère hydrosoluble et non peptidique est lié covalentement au rétinoïde par une liaison hydrolitiquement stable.

21. Composition selon la revendication 1, dans laquelle ledit polymère hydrosoluble et non peptidique a un poids moléculaire moyen de 500 daltons à 100 000 daltons.

22. Composition selon la revendication 21, dans laquelle ledit polymère hydrosoluble et non peptidique a un poids moléculaire moyen de 750 daltons à 40 000 daltons.

23. Composition selon la revendication 2, dans laquelle ledit polyéthylèneglycol est à extrémité protégée.

24. Composition selon la revendication 2, dans laquelle ledit polyéthylèneglycol est à extrémité protégée par un groupe alkoxy.

25. Composition selon la revendication 2, dans laquelle le polyéthylèneglycol est sélectionné parmi le groupe comprenant le polyéthylèneglycol linéaire, le polyéthylèneglycol ramifié, le polyéthylèneglycol fourché et le polyéthylèneglycol en haltère.

26. Composition selon la revendication 1 moins un agent requis pour la solubilisation dudit rétinoïde dans un excipient vecteur.

27. Composition conjuguée polymère-rétinoïde de l'une quelconque des revendications 1 à 26, à utiliser en tant que médicament administré par voie pulmonaire à un sujet humain.

28. Composition selon la revendication 27 pour le traitement de la bronchopneumopathie chronique obstructive.

29. Utilisation d'une composition conjuguée polymère-rétinoïde de l'une quelconque des revendications 1 à 26, pour la préparation d'un médicament pour traiter la bronchopneumopathie chronique obstructive, ledit médicament étant administré par inhalation.

30. Conjugué polymère-rétinoïde selon la revendication 18, dans lequel ledit polymère est lié covalentement, par une liaison hydrolytiquement dégradable, au carbone carbonyle de l'acide rétinoïque et, en outre, dans lequel (i) si le polymère est un polyéthylèneglycol linéaire et si la liaison est une liaison ester, alors le poids moléculaire du polyéthylèneglycol est d'au moins 2000 daltons, et (ii) si le polymère est un polyéthylèneglycol linéaire à extrémité protégée et si la liaison est une liaison amide, alors le polyéthylèneglycol a un poids moléculaire d'au moins 5000 daltons.

31. Conjugué polymère-rétinoïde selon la revendication 30, dans lequel ladite liaison hydrolytiquement dégradable est sélectionnée parmi le groupe comprenant le carboxylate-ester, l'amide et le thiolester.

32. Conjugué polymère-rétinoïde selon la revendication 30, dans lequel le conjugué polymère-rétinoïde comprend la structure : 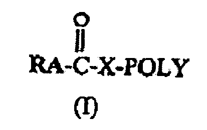 dans laquelle RA, avec le groupe carbonyle, représente un fragment de molécule d'acide rétinoïque,
X est un hétéroatome sélectionné parmi O, N-H et S, et
POLY est le polymère hydrosoluble non peptidique,
dans laquelle (i) si le POLY est un polyéthylèneglycol linéaire et X est un O, alors le poids moléculaire du POLY est d'au moins 2000 daltons, et (ii) si POLY est un polyéthylèneglcyol linéaire à extrémité protégée et X est un N, alors POLY a un poids moléculaire d'au moins 5000 daltons.

33. Conjugué polymère-rétinoïde selon la revendication 32 sélectionné parmi le groupe comprenant :

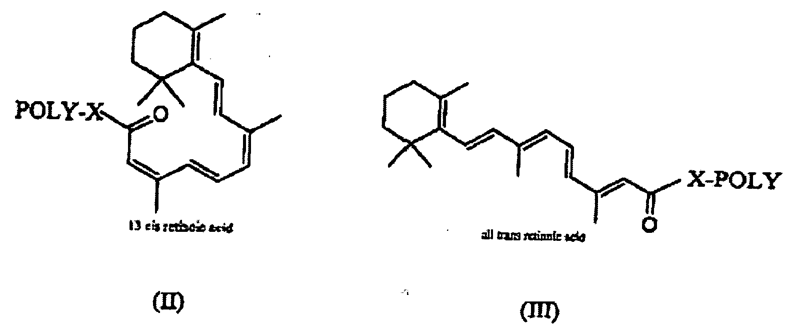

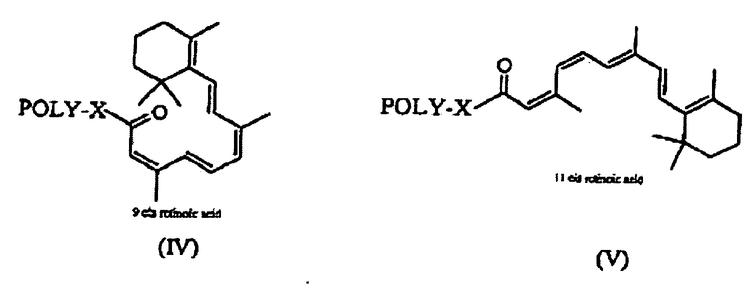

34. Conjugué polymère-rétinoïde selon la revendication 33, dans lequel POLY est un poly(éthylèneglycol).

35. Conjugué polymère-rétinoïde selon la revendication 33, dans lequel X est O ou S.

36. Conjugué polymère-rétinoïde selon la revendication 33, dans lequel POLY possède 2 à 300 terminus.

37. Conjugué polymère-rétinoïde selon la revendication 33, dans lequel ledit POLY est à extrémité protégée.

38. Conjugué polymère-rétinoïde selon la revendication 37, ayant la structure suivante : 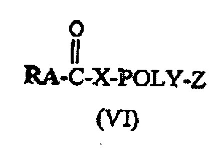 dans laquelle Z est un groupe fonctionnel.

39. Conjugué polymère-rétinoïde selon la revendication 38, dans lequel Z a la structure 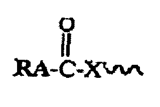 dans laquelle X', avec le carbonyle, est une liaison hydrolytiquement dégradable et 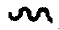 représente le point d'attachement à POLY.

40. Conjugué polymére-rétinoïde selon la revendication 38, dans lequel POLY est le poly(éthylèneglycol) et Z est méthoxy.

41. Conjugué polymère-rétinoïde selon la revendication 33 ayant la structure : 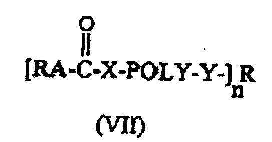 dans laquelle :
n est un nombre entier compris entre 3 et 100 ;
R est une principale molécule centrale ;
X, avec le groupe carbonyle adjacent, est une liaison hydrolytiquement dégradable ;
Y est une liaison ; et
chaque POLY est un polymère hydrosoluble et non peptidique sélectionné indépendamment, tel que défini à la revendication 1.

42. Conjugué polymère-rétinoïde selon la revendication 41, dans lequel n est compris entre 3 et 20.

43. Conjugué polymère-rétinoïde selon la revendication 41, dans lequel Y est O, S ou NH.

44. Conjugué polymère-rétinoïde selon la revendication 41, dans lequel R est un résidu d'une principale molécule centrale sélectionné parmi le groupe comprenant le glycérol, les oligomères du glycérol, le pentaérythritol, le sorbitol et la lysine.

45. Conjugué polymère-rétinoïde selon la revendication 32, dans lequel POLY est un polyéthylèneglycol sélectionné parmi le groupe comprenant le PEG linéaire, ramifié, fourché ou en forme d'haltère.

46. Méthode de préparation d'une formule rétinoïde convenant à l'administration par voie pulmonaire, ladite méthode comprenant les étapes qui consistent à :
lier covalentement un rétinoïde et un polymère hydrosoluble, tel que recité dans la revendication 1, pour former un conjugué polymère-rétinoïde hydrosoluble,
préparer une composition pharmaceutique comprenant ledit conjugué, dans laquelle ladite composition convient à l'administration par voie pulmonaire, et
l'aérosolisation de ladite composition.

47. Méthode selon la revendication 46, dans laquelle ladite étape de préparation comprend l'association entre ledit conjugué et un ou plusieurs excipients.
